(19) **Europäisches Patentamt / European Patent Office / Office européen des brevets**

(11) **EP 3 685 739 B1**

(12) # EUROPEAN PATENT SPECIFICATION

(45) Date of publication and mention
of the grant of the patent:
**11.02.2026 Bulletin 2026/07**

(51) International Patent Classification (IPC):
**A61B 5/327** (2021.01)   **A61B 5/341** (2021.01)
**A61B 5/344** (2021.01)

(21) Application number: **19215454.0**

(22) Date of filing: **12.12.2019**

(52) Cooperative Patent Classification (CPC):
**A61B 5/7267; A61B 5/341; A61B 5/344;**
**A61B 8/0866; G16H 30/40; G16H 50/20;**
A61B 2503/02; G16H 20/40

(54) **DEVICE FOR CLASSIFYING FETAL ECG**

VORRICHTUNG ZUR KLASSIFIZIERUNG VON FETALEN EKGS

DISPOSITIF DE CLASSIFICATION D'ECG FETAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.01.2019 EP 19150918**

(43) Date of publication of application:
**29.07.2020 Bulletin 2020/31**

(73) Proprietor: **Nemo Healthcare B.V.
5503 LL Veldhoven (NL)**

(72) Inventor: **VULLINGS, Rik
5504 DB Veldhoven (NL)**

(74) Representative: **DeltaPatents B.V.
Fellenoord 370
5611 ZL Eindhoven (NL)**

(56) References cited:
**US-A1- 2010 185 108**

• VULLINGS R ET AL: "Vectorcardiographic loop alignment for fetal movement detection using the Expectation-Maximization algorithm and Support Vector Machines", ENGINEERING IN MEDICINE AND BIOLOGY SOCIETY (EMBC), 2013 34TH ANNUAL INTERNATIONAL CONFERENCE OF THE IEEE, IEEE, 3 July 2013 (2013-07-03), pages 2915 - 2918, XP032489483, ISSN: 1557-170X, [retrieved on 20130925], DOI: 10.1109/EMBC.2013.6610150

• SUNEMARK M ET AL: "Serial VCG/ECG Analysis Using Neural Networks", vol. 31, no. 1, 25 May 2002 (2002-05-25), pages 59 - 69, XP029582583, ISSN: 0010-4809, Retrieved from the Internet <URL:https://www.sciencedirect.com/science/article/pii/S0010480997914622?via%3Dihub> DOI: 10.1006/CBMR.1997.1462

• ALIREZA MEHRI DEHNAVI ET AL: "Detection and classification of cardiac ischmia using vectorcardigram signal via neural network", JOURNAL OF RESEARCH IN MEDICAL SCIENCES, 1 February 2011 (2011-02-01), XP055489891, Retrieved from the Internet <URL:https://www.sciencedirect.com/science/article/pii/S0010480997914622?via%3Dihub> [retrieved on 20180703]

• WARMERDAM G ET AL: "QRS classification and spatial combination for robust heart rate detection in low-quality fetal ECG recordings", ENGINEERING IN MEDICINE AND BIOLOGY SOCIETY (EMBC), 2013 34TH ANNUAL INTERNATIONAL CONFERENCE OF THE IEEE, IEEE, 3 July 2013 (2013-07-03), pages 2004 - 2007, XP032489537, ISSN: 1557-170X, [retrieved on 20130925], DOI: 10.1109/EMBC.2013.6609923

• VULLINGS RIK: "Fetal Electrocardiography and Deep Learning for Prenatal Detection of Congenital Heart Disease", 2019 COMPUTING IN CARDIOLOGY (CINC), CREATIVE COMMONS, 8 September 2019 (2019-09-08), XP033720830, DOI: 10.23919/CINC49843.2019.9005870

EP 3 685 739 B1

• **VULLINGS R ET AL: "Bayesian Approach to Patient-Tailored Vectorcardiography", IEEE TRANSACTIONS ON BIOMEDICAL ENGINEERING, IEEE, USA, vol. 57, no. 3, 1 March 2010 (2010-03-01), pages 586 - 595, XP011283728, ISSN: 0018-9294**

## Description

### FIELD OF THE INVENTION

The invention relates to a device for processing a fetal electrocardiogram, a device for training a machine learning classifier, a method for processing a fetal electrocardiogram, a method for training a machine learning classifier, and a computer readable medium.

### BACKGROUND

Congenital heart disease (CHD) is the most common severe congenital anomaly worldwide. With a reported incidence of eight per 1000 births', around 1.35 million newborns are born with CHD every year[2]. A third of these defects are critical[1,3,4]. CHD is associated with significant mortality and long-term morbidity and is responsible for more than half of the deaths from congenital anomalies in infancy[5]. Approximately 4.5% of fetuses with CHD die in-utero and 21% die postnatally[6]. Additionally, those who survive with CHD have a nine-fold increased risk of intellectual disability[7].

The timely prenatal detection of CHD has some important advantages. In the case of severe defects, parents may choose to terminate the pregnancy. This decision may also be influenced by the presence of associated chromosomal and extracardiac anomalies which should also be looked for, as up to 26% of fetuses with CHD have chromosomal anomalies and 23% have extracardiac anomalies[8]. When the pregnancy is continued, a prenatal diagnosis of CHD allows the parents time to prepare for the arrival of a potentially sick child. Furthermore, it facilitates appropriate changes in obstetric and neonatal management, including intra-uterine therapy, planning of the delivery in a center with the required neonatal and cardiothoracic surgical care facilities, and timely treatment after birth. It has been demonstrated that prenatal diagnosis of CHD increases survival rates and decreases long-term morbidity[9-18].

The antenatal detection of CHD is currently done via ultrasonic examination. In most developed countries, a standard anomaly screening is performed at around 20 weeks of gestation. The assessment of the fetal heart is an important component of this screening. Despite mass screening, many cases of CHD are still missed. In the Netherlands, fetal cardiac screening during the second trimester was standardized and introduced in 2007. With an extensive training program, 59.7% of severe CHD and 44% of isolated severe CHD were detected antenatally[19]. Though this detection rate is much higher than that reported in most Western countries[20-25], it still leaves room for further improvement.

Vullings R et al.: "Vectorcardiographic loop alignment for fetal movement detection using the Expectation-Maximization algorithm and Support Vector Machines", DOI: 10.1109/EMBC.2013.6610150, discloses detection of fetal movement based on VCG loop alignment. Sunemark M et al.: "Serial VCG/ECG Analysis Using Neural Networks", DOI: 10.1006/CBMR.1997.1462 discloses applying neural networks to VCG/ECG measurements.

The calculation of fetal VCGs from multiple fetal ECGs and early-stage diagnosis of congenital heart diseases (CHG) from fetal VCGs is suggested in VULLINGS R ET AL: "Bayesian Approach to Patient-Tailored Vectorcardiography", IEEE TRANSACTIONS ON BIOMEDICAL ENGINEERING, IEEE, USA, vol. 57, no. 3, March 2010, pages 586-595, ISSN: 0018-9294, DOI: 10.1109/TBME.2009.2033664.

The following references are given as background, and for the reasons give herein.

1. Dolk H, Loane M, Garne E, a European Surveillance of Congenital Anomalies (EUROCAT) Working Group. Congenital Heart Defects in Europe: Prevalence and Perinatal Mortality, 2000 to 2005. Circulation. 1 maart 2011;123(8):841-9.

2. van der Linde D, Konings EEM, Slager MA, Witsenburg M, Helbing WA, Takkenberg JJM, e.a. Birth Prevalence of Congenital Heart Disease Worldwide. J Am Coll Cardiol. november 2011;58(21):2241-7.

3. Hoffman JIE, Kaplan S. The incidence of congenital heart disease. J Am Coll Cardiol. 19 juni 2002;39(12):1890-900.

4. Nelle M, Raio L, Pavlovic M, Carrel T, Surbek D, Meyer-Wittkopf M. Prenatal diagnosis and treatment planning of congenital heart defects-possibilities and limits. World J Pediatr. februari 2009;5(1):18-22.

5. Young ID, Clarke M. Lethal malformations and perinatal mortality: a 10 year review with comparison of ethnic differences. Br Med J Clin Res Ed. 11 juli 1987;295(6590):89-91.

6. Fesslova' V, Brankovic J, Boschetto C, Masini A, Prandstraller D, Perolo A, e.a. Changed outcomes of fetuses with congenital heart disease: new Italian Multicentre study. J Cardiovasc Med. augustus 2015;16(8):568-75.

7. Razzaghi H, Oster M, Reefhuis J. Long-Term Outcomes in Children with Congenital Heart Disease: National Health Interview Survey. J Pediatr. januari 2015;166(1):119-124.e1.

8. Clur SAB, Van Brussel PM, Mathijssen IB, Pajkrt E, Ottenkamp J, Bilardo CM. Audit of 10 years of referrals for fetal echocardiography: AUDIT OF 10 YEARS OF REFERRALS FOR FETAL ECHOCARDIOGRAPHY. Prenat Diagn. september 2011;n/a-n/a.

9. Bonnet D, Coltri A, Butera G, Fermont L, Le Bidois J, Kachaner J, e.a. Detection of transposition of the great arteries in fetuses reduces neonatal morbidity and mortality. Circulation. 23 februari 1999;99(7):916-8.

10. Mahle WT, Clancy RR, McGaurn SP, Goin JE, Clark BJ. Impact of prenatal diagnosis on survival and early neurologic morbidity in neonates with the hypoplastic left heart syndrome. Pediatrics. juni 2001;107(6):1277-82.

11. Tworetzky W, McElhinney DB, Reddy VM, Brook MM, Hanley FL, Silverman NH. Improved surgical outcome after fetal diagnosis of hypoplastic left heart syndrome. Circulation. 6 maart 2001;103(9):1269-73.

12. Franklin O, Burch M, Manning N, Sleeman K, Gould S, Archer N. Prenatal diagnosis of coarctation of the aorta improves survival and reduces morbidity. Heart Br Card Soc. januari 2002;87(1):67-9.

13. Mäkikallio K, McElhinney DB, Levine JC, Marx GR, Colan SD, Marshall AC, e.a. Fetal aortic valve stenosis and the evolution of hypoplastic left heart syndrome: patient selection for fetal intervention. Circulation. 21 maart 2006;113(11):1401-5.

14. Kaguelidou F, Fermont L, Boudjemline Y, Le Bidois J, Batisse A, Bonnet D. Foetal echocardiographic assessment of tetralogy of Fallot and post-natal outcome. Eur Heart J. 18 januari 2008;29(11):1432-8.

15. Trines J, Fruitman D, Zuo KJ, Smallhorn JF, Hornberger LK, Mackie AS. Effectiveness of Prenatal Screening for Congenital Heart Disease: Assessment in a Jurisdiction With Universal Access to Health Care. Can J Cardiol. juli 2013;29(7):879-85.

16. Hunter LE, Simpson JM. Prenatal screening for structural congenital heart disease. Nat Rev Cardiol. juni 2014;11(6):323-34.

17. van Velzen CL, Haak MC, Reijnders G, Rijlaarsdam MEB, Bax CJ, Pajkrt E, e.a. Prenatal detection of transposition of the great arteries reduces mortality and morbidity. Ultrasound Obstet Gynecol. maart 2015;45(3):320-5.

18. Brown KL, Ridout DA, Hoskote A, Verhulst L, Ricci M, Bull C. Delayed diagnosis of congenital heart disease worsens preoperative condition and outcome of surgery in neonates. Heart Br Card Soc. september 2006;92(9):1298-302.

19. van Velzen C, Clur S, Rijlaarsdam M, Bax C, Pajkrt E, Heymans M, e.a. Prenatal detection of congenital heart disease-results of a national screening programme. BJOG Int J Obstet Gynaecol. februari 2016;123(3):400-7.

20. Levy DJ, Pretorius DH, Rothman A, Gonzales M, Rao C, Nunes ME, e.a. Improved Prenatal Detection of Congenital Heart Disease in an Integrated Health Care System. Pediatr Cardiol. maart 2013;34(3):670-9.

21. Marek J, Tomek V, Skovranek J, Povysilova V, Samanek M. Prenatal ultrasound screening of congenital heart disease in an unselected national population: a 21-year experience. Heart. 15 januari 2011;97(2):124-30.

22. Buskens E, Grobbee DE, Frohn-Mulder IM, Stewart PA, Juttmann RE, Wladimiroff JW, e.a. Efficacy of routine fetal ultrasound screening for congenital heart disease in normal pregnancy. Circulation. 1 juli 1996;94(1):67-72.

23. Sharland GK, Allan LD. Screening for congenital heart disease prenatally. Results of a 2 1/2-year study in the South East Thames Region. Br J Obstet Gynaecol. maart 1992;99(3):220-5.

24. Stümpflen I, Stümpflen A, Wimmer M, Bernaschek G. Effect of detailed fetal echocardiography as part of routine prenatal ultrasonographic screening on detection of congenital heart disease. Lancet Lond Engl. 28 september 1996;348(9031):854-7.

25. Galindo A, Herraiz I, Escribano D, Lora D, Melchor JC, de la Cruz J. Prenatal Detection of Congenital Heart Defects: A Survey on Clinical Practice in Spain. Fetal Diagn Ther. 2011;29(4):287-95.

26. Van Laar J, Warmerdam G, Verdurmen K, Vullings R, Peters C, Houterman S, Wijn P, Andriessen P, Van Pul C, Oei G. Fetal heart rate variability during pregnancy, obtained from non-invasive electrocardiogram recordings. Acta Obstet Gynecol Scand. 2014;93:93-101.

27. Verdurmen K, Lempersz C, Vullings R, Schroer C, Delhaas T, Van Laar J, Oei S. Normal ranges for fetal electrocardiogram values for the healthy fetus of 18-24 weeks of gestation: a prospective cohort study. BMC Pregnancy Childbirth. 2016;16:227.

28. Vullings R, Peters CHL, Sluijter RJ, Mischi M, Oei SG, Bergmans JWM. Dynamic segmentation and linear prediction for maternal ECG removal in antenatal abdominal recordings. Physiol Meas. 1 maart 2009;30(3):291-307.

29. Vullings R, Peters CHL, Mossavat I, Oei SG, Bergmans JWM. Bayesian Approach to Patient-Tailored Vectorcardiography. IEEE Trans Biomed Eng. maart 2010;57(3):586-95.

30. Warmerdam GJJ, Vullings R, Schmitt L, Van Laar JOEH, Bergmans JWM. A Fixed-Lag Kalman Smoother to Filter Power Line Interference in Electrocardiogram Recordings. IEEE Trans Biomed Eng. augustus 2017;64(8):1852-61.

31. Warmerdam GJJ, Vullings R, Schmitt L, Van Laar JOEH, Bergmans JWM. Hierarchical Probabilistic Framework for Fetal R-Peak Detection, Using ECG Waveform and Heart Rate Information. IEEE Trans Signal Process. 15 augustus 2018;66(16):4388-97.

32. Fotiadou E, van Laar JOEH, Oei SG, Vullings R. Enhancement of low-quality fetal electrocardiogram based on time-sequenced adaptive filtering. Med Biol Eng Comput [Internet]. 25 juni 2018 [geciteerd 2 oktober 2018]; Available at: http://link.springer.com/10.1007/s11517-018-1862-8

33. Vullings R, Mischi M, Oei SG, Bergmans JWM. Novel Bayesian Vectorcardiographic Loop Alignment for Improved Monitoring of ECG and Fetal Movement. IEEE Trans Biomed Eng. juni 2013;60(6):1580-8.

34. Verdurmen KMJ, Hulsenboom ADJ, van Laar JOEH, Wijn PFF, Vullings R, Oei SG. Orientation of the electrical heart axis in mid-term pregnancy. Eur J Obstet Gynecol Reprod Biol. december 2016;207:243-6.

## SUMMARY OF THE INVENTION

Improvement of CHD detection rates are obtained from the use of non-invasive fetal electrocardiography. In fetal electrocardiography it is possible to acquire a high-quality fetal electrocardiogram (fECG) at gestational ages of about 18 weeks and higher[26,27]. However, no easy applicable guidelines are known that indicate to a medical operator what constitutes a normal fetal ECG, say, around 20 weeks of gestation. Without knowing what is normal, it is very hard to draw conclusions about what might be an abnormal ECG and what ECG abnormalities might be associated with CHD. Screening for CHD during pregnancy using ECG signals may be done before or after 20 weeks of gestation, e.g., between 18 and 24 weeks of gestation.

There is a need for a device which can assist fetal electrocardiogram classification. It is not needed that such a device has a perfect CHD detection rate. Rather, classifying children which may be further screened after birth of before birth in a specialized center will improve CHD care even if some children are incorrectly classified. Note that in specialized hospitals, in most cases, a correct diagnosis can be made by an experienced pediatric cardiologist using high-end ultrasound equipment. However, for screening purposes such a solution is not suitable, as it would be far too expensive. If the detection rate goes up without too many false positives, e.g., incorrect CHD detections, then the care can be improved, without too much extra costs. Sending more children to special centers leads to higher costs but these can be recovered, if it prevents even greater costs associated with undetected CHD.

A device for classifying fECGs according to the invention is presented in claim 1. Furthermore, a device for training the classifying device is disclosed. The dependent claims define advantageous embodiments. These devices are electronic devices, for example, a computer, an ECG console, and the like. The electronic classification and training methods described herein may be applied to reduce the number of children with undetected CHD.

An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium.

## BRIEF DESCRIPTION OF THE DRAWINGS

Further details, aspects, and embodiments of the invention will be described, by way of example only, with reference to the drawings. Elements in the figures are illustrated for simplicity and clarity and have not necessarily been drawn to scale. In the Figures, elements which correspond to elements already described may have the same reference numerals. In the drawings,

   Figure 1a schematically shows an example of an embodiment of a device for processing a fetal electrocardiogram,
   Figure 1b schematically shows an example of an embodiment of a device for training a machine learning classifier for classifying a fetal vector cardiogram,
   Figure 1c schematically shows an example of an embodiment of a storage for storing trained parameters for a machine learning classifier,
   Figure 2a schematically shows an example of an embodiment of a device for processing a fetal electrocardiogram,
   Figure 2b schematically shows an example of an embodiment of a device for processing a fetal electrocardiogram,
   Figure 3 schematically shows an example of an embodiment of a device for training a machine learning classifier for classifying a fetal vector cardiogram,
   Figure 4 schematically shows examples of embodiments of training data,
   Figure 5 schematically shows an example of an embodiment of a method of processing a fetal electrocardiogram,
   Figure 6 schematically shows an example of an embodiment of a neural network for classifying a fetal vector cardiogram,
   Figure 7 schematically shows an example of sensitivity for an embodiment of the device for processing a fetal electrocardiogram,
   Figure 8 schematically shows an example of an embodiment of a device for inputting fetal orientation,
   Figure 9a schematically shows an example of a histogram of fetal orientation for fetuses without CHD.
   Figure 9b schematically shows an example of a histogram of fetal orientation for fetuses with CHD,

Figure 10 schematically shows an example of an embodiment of a method for processing a fetal electrocardiogram,
Figure 11 schematically shows an example of an embodiment of a method for training a machine learning classifier for classifying a fetal vector cardiogram,
Figure 12a schematically shows a computer readable medium having a writable part comprising a computer program according to an embodiment,
Figure 12b schematically shows a representation of a processor system according to an embodiment.

List of Reference Numerals:

| | |
|---|---|
| 110 | a device for processing a fetal electrocardiogram |
| 111 | a device for training a machine learning classifier for classifying a fetal vector cardiogram |
| 112 | a storage for storing trained parameters for a machine learning classifier |
| 130, 131 | a communication interface |
| 140, 141 | a processor |
| 150, 151 | a memory |
| 200, 201 | a device for processing a fetal electrocardiogram |
| 210 | a signal input |
| 211 | multiple electrodes |
| 220, 221 | a storage for storing trained parameters for a machine learning classifier |
| 230 | a fetal ECG unit |
| 240 | a vector cardiogram unit |
| 250 | a processing unit |
| 260, 261 | a machine learning classifier |
| 270 | an output unit |
| 280 | an orientation input |
| 281 | an orientation input device |
| 300 | a device for training a machine learning classifier for classifying a fetal vector cardiogram |
| 310 | a storage for storing a training data |
| 320 | a storage for storing parameters for a machine learning classifier |
| 350 | a training data processing unit |
| 360 | a machine learning unit |
| 410 | a training data |
| 412 | a vector cardiogram |
| 419 | a classification |
| 420 | a training data |
| 422-424 | a vector cardiogram |
| 429 | a classification |
| 430 | a training data |
| 432 | a vector cardiogram |
| 434 | an orientation |
| 439 | a classification |
| 1000 | a computer readable medium |
| 1010 | a writable part |
| 1020 | a computer program |
| 1110 | integrated circuit(s) |
| 1120 | a processing unit |
| 1122 | a memory |
| 1124 | a dedicated integrated circuit |
| 1126 | a communication element |
| 1130 | an interconnect |
| 1140 | a processor system |

## DETAILED DESCRIPTION OF EMBODIMENTS

While this invention is susceptible of embodiment in many different forms, there are shown in the drawings and will herein be described in detail one or more specific embodiments, with the understanding that the present disclosure is to be considered as exemplary of the principles of the invention and not intended to limit the invention to the specific embodiments shown and described.

In the following, for the sake of understanding, elements of embodiments are described in operation. However, it will be apparent that the respective elements are arranged to perform the functions being described as performed by them.

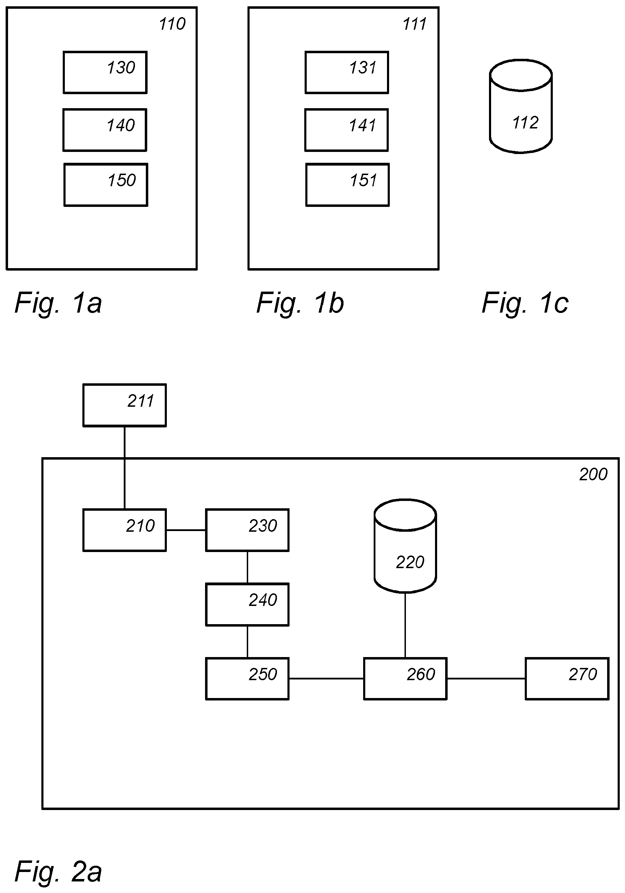

**Figure 1a** schematically shows an example of an embodiment of a device for processing a fetal electrocardiogram. **Figure 1b** schematically shows an example of an embodiment of a device for training a machine learning classifier for classifying a fetal vector cardiogram. **Figure 1c** schematically shows an example of an embodiment of a storage for storing trained parameters for a machine learning classifier.

Device 110 comprises a communication interface 130, a processor 140, and a memory 150. Device 111 comprises a communication interface 131, a processor 141, and a memory 151.

The various devices 110, 111 and 112 may communicate with each other over a computer network. The computer network may be an internet, an intranet, a LAN, a WLAN, etc. The computer network may be the Internet. The devices comprise a communication interface which is arranged to communicate with other devices as needed. For example, the communication interface may comprise a connector, e.g., a wired connector, e.g., an Ethernet connector, an optical connector, etc., or a wireless connector, e.g., an antenna, e.g., a Wi-Fi, 4G or 5G antenna. For example, device 110 may comprise a communication interface 130 configured to receive input signal such as electrocardiogram signals and/or orientation signals, or trained parameters and the like. For example, communication interface 130 may be configured to report classification results to a user, e.g., on a display connectable to device 110. For example, device 111 may comprise a communication interface 131 to receive training data, and/or to send trained parameters for a machine learning classifier. Storage 112 may be an external storage, e.g., external to devices 110 and/or 111. For example, storage 112 may be a cloud storage. For example, storage 112 may be an internal storage, e.g., from one or both of devices 110 and 111. Training data, signal inputs and trained parameters may be digital data, e.g., received in electronic form.

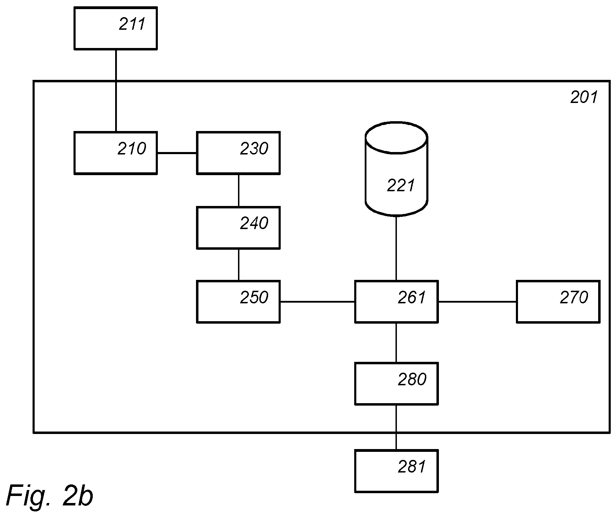

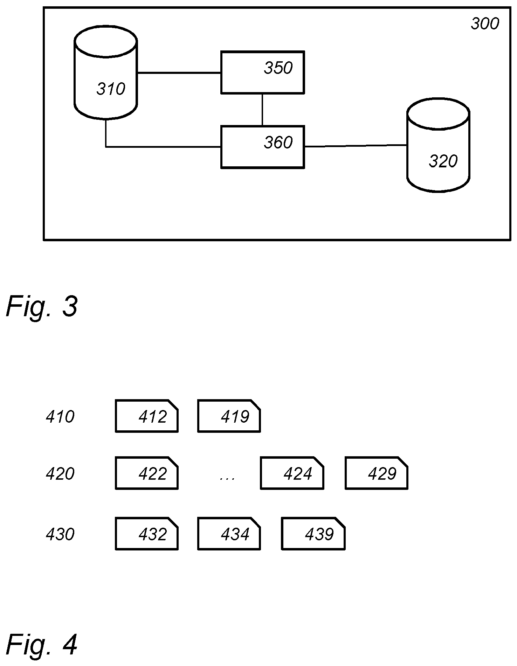

The execution of device 110 and 111 is implemented in a processor circuit, examples of which are shown herein. Figures 2a, 2b and 3 show functional units that may be functional units of the processor circuit. For example, these figures may be used as a blueprint of a possible functional organization of the processor circuit. The processor circuit is not shown separate from the units in these figures. For example, the functional units shown in these figures may be wholly or partially implemented in computer instructions that are stored at device 110 or 111, e.g., in electronic memory 150 or 151 of device 110 or 111, and are executable by a microprocessor of device 110 or 111 In hybrid embodiments, functional units may be implemented partially in hardware, e.g., as coprocessors, e.g., signal coprocessors, and partially in software stored and executed on device 110 and/or 111.

**Figure 2a** schematically shows an example of an embodiment of a device 200 for processing a fetal electrocardiogram.

Classifying device 200 comprises a signal input 210. Signal input 210 is configured to receive multiple ECG signals from multiple electrodes 211. The multiple electrodes are configured for arranging on the abdomen of a pregnant woman. For example, during use multiple electrodes 211 are arranged on the abdomen of a pregnant woman, say at a gestation of about 20 weeks. Screening may be done earlier or later. The number of multiple electrodes 211 may be, say, three or more, four or more, six or more, etc. Preferably, the electrodes are applied in a fixed arrangement. For example, the electrodes may be connected with each other using half-flexible connections so as to indicate the desired distance between electrodes. For example, ECG signals may be recorded by device 200 for some set amount of time, say 5 minutes, 10 minutes, or the like. The multiple electrodes 211 record one or more heart-beats of the fetus and one or more unrelated signals that obscure the fetal ECG, in particular the mothers ECG. To this end classifying device 200 comprises a fetal ECG unit 230 which is configured to obtain at least a partial fetal ECG signal from the multiple ECG signals. For example, fECG unit 230 may be configured to filter-out the mother's ECG signal from the signal received at input 210 to obtain the fECG. In particular, fECG unit 230 may be configured to obtain multiple fECG signals from the multiple ECG signal received from multiple electrodes 211. fECG unit 230 may be configured to perform further processing on the multiple fECGs. For example, fECG unit 230 may be configured to segment the multiple fECGs into individual heartbeats, e.g., by detecting so-called QRS complexes in the signal. Averaging provides a significant improvement in signal quality, and may be done at the ECG stage, e.g., in fECG unit 230, e.g., after filtering out the confounding signals, or at the VCG state, e.g., in unit 240 discussed below.

In an embodiment, device 200, e.g., fECG unit 230 may be configured to detect fetal QRS complexes in the fetal ECG signals, and to reject the classification if the number of detected QRS complexes is below a threshold. This may be an important precaution, since in any case the processing of fetal ECGs is done on an inherently noisy signal. By detecting early in the processing if the quality of the obtained fECG signals is too low, the process may be aborted. Without this precaution the process could continue as usual, however it is to be expected that the eventual classification is as much a product of noise as of anything else. The threshold may be set empirically, e.g., by keeping track of the quality of classification with respect to the number of detected QRS complexes. For example, one may set the rejection threshold at 25 QRS complexes or less, 50 QRS complexes or less, etc. If a classification is rejected then this may be reported, e.g., to an operator of device 200. For example, obtaining the ECG signals from electrodes 211 may be repeated, possibly at a later date, etc. Accordingly, this feature protects against an unreliable classification.

Classifying device 200 may further comprise a vector cardiogram unit 240. VCG unit 240 is configured to calculate at least one fetal vector cardiogram (VCG) from said multiple fetal ECG signals. For example, a number of QRS complexes are selected from the multiple fECG signals that correspond to the same fetal heartbeat and an fVCG is computed for that heartbeat. Calculating a VCG may be done using a linear operator. In particular, the fetal VCG may be obtained from a linear combination of fetal ECG complexes. This linear combination is in particular determined by the position of the electrodes. Note that, the fetus may be closer to a one electrode than to another, so that the attenuation that occurs for each fECG during its conduction of the fetal heart to the electrode may be different from different fECGs. In an embodiment, device 200, e.g., unit 240, is configured to estimate this attenuation and compensate for it. This estimate is a non-linear process. Because of this a different result will be obtained if fetal VCGs are averaged or fetal ECGs, the former being preferred; averaging of fetal VCGs is further expanded upon below.

Estimating the attenuation and compensating for it, can be done in a number of ways. For example, this may be done with a Bayes method. In this approach, both the VCG and the attenuation are iteratively estimated. This will cause attenuation and VCG to converge, so that both attenuation and VCG are found.

In an embodiment, an improved approach is used. Here a non-linear minimization problem with constraints is created. The underlying model remains the same: ECG = scaling x projection x VCG, wherein the scaling depends on attenuation and projection of electrode position. See Eq (3) in the paper [29] wherein V = ECG, alpha = scaling, D is electrode positions, S is VCG. In the Bayes method, the scaling/attenuation can become negative which is undesirable. In the improved approach, the following minimization may be defined: $\hat{V} = \alpha D(D'D)^{-1}D'\alpha^{-1}V$

Matrix transposition is denoted with an apostrophe. In this way, one can calculate a VCG from the ECG V, e.g., using $S = (D'D)^{-1}D'\alpha^{-1}V$ and from this VCG again the ECG, e.g., using $\alpha DS$. The calculated ECG $\hat{V}$ should resemble as much as possible the actual ECG V. In short, this method calculates the scaling $\alpha$ such that it minimizes the difference between $\hat{V}$ and $V$, under the constraints that the elements of the diagonal matrix $\alpha$ are positive and that in addition the average of $\alpha$ is equal to 1. The latter prevents the solution from converging to very large or small values.

If there is too much noise on the fECG, this may be resolved by averaging fECGs that correspond to multiple heartbeats before computing the VCG. However, if noise allows then fetal VCGs may be computed that correspond to a single heartbeat, which may then in turn be averaged. One way to reduce noise without averaging over multiple heart beats is by using an adaptive filter; see, e.g., "Enhancement of low-quality fetal electrocardiogram based on time-sequenced adaptive filtering", by Fotiadou, et al. Adaptive filtering may advantageously be combined with averaging.

In an embodiment, a VCG may be represented as a 3D array of vectors. For example, a VCG may be represented as a 3x1024 array. The array may be larger or smaller. Each element in this array may represent a voltage. In an embodiment, the entries in the array may be expressed in a double format, e.g., 64 bit floating points. Entries may use more or fewer bits, e.g., 16 bits, 32 bits, etc.

In an embodiment, a VCG is sampled at 2 kHz. A heartbeat of a fetus typically lasts about 0.45s, which amounts to 900 samples. For some children this is longer, for others shorter. This may be resolved by padding, e.g., zero-padding, and/or truncating the array to make sure all VCGs get a length of 1024. This is convenient as the machine learning classifier may then be configured to receive a fixed length array.

According to the invention, computing the fetal VCGs is performed on a higher sample-rate, after which the fetal vector cardiogram is down-sampled to a lower sample-rate before providing as an input to the machine learning classifier. For example, attenuation compensation, alignment, and/or averaging may be done at a higher sampling rate, after which the final VCG is down sampled before providing it to the classifier. A higher accuracy during pre-processing of the VCGs is beneficial to obtain a better quality input for the classifier. In an embodiment, one may use for example, a sampling rate that is twice as high or more during pre-processing than used at the input of the classifier. It is noted however, that this is not necessary; in an embodiment, sampling rate during pre-processing may be the same as used at the input of the classifier. The same holds for the resolution. During pre-processing the VCG elements may be represented with more bits, say, twice or more bits, than at the input of the classifier. Padding and/or truncating may be done at the input of the classifier, e.g., after pre-processing so that during pre-processing a variable length array may be used. Down-sampling is thus not necessary, embodiment may avoid down-sampling before the neural network, e.g., compute at a constant sampling rate, or may even up-sample before the neural network.

In an embodiment, first interferences are suppressed in the raw ECG signals, such as the maternal ECG, powerline, and electromyographic signals from within the maternal body, e.g., using filtering, e.g., using adaptive filtering, e.g., adaptive template subtraction, adaptive Kalman filtering, etc. The fetal QRS complexes may then be identified. Subsequently, the fetal ECG signals may be segmented, e.g., in such a way that every segment contains exactly one heartbeat and so that the segments are synchronized, e.g., on the position of the R-peak. From the fetal ECGs a vector cardiogram (VCG) of the fetus can be calculated. As pointed out signal quality can be improved by averaging. For example, the fetal ECGs may be enhanced by averaging multiple segments, e.g., at least 10, at least 30, etc. This procedure may be performed for each of the channels of fetal ECG data, e.g., each of six channels. Preferably, averaging is done at the VCG stage, though. A VCG entails a three-dimensional representation of the fetal electrical cardiac activity and may be calculated for multiple heartbeats, or for multiple averaged heartbeats. A fetal VCG may be determined in the xyz-coordinate system that is described with respect to the maternal body. In the VCGs one may detect and correct for fetal movements during the ECG measurement. Ultrasound assessments during the measurements may be used to correct for cumulative errors in this movement correction method and to determine the initial orientation of the fetus. After correcting for fetal movements, the VCGs may be averaged to further enhance signal quality. It turns out in practice though, that error due to movement is small, and that correcting for fetal movements, e.g., using ultrasound is not required. Ultrasound is one way to determine fetal orientation.

In an embodiment, classifier device 200 comprises a processing unit 250. Processing unit 250 is optional, but may provide an important contribution to the accuracy of device 200. For example, in an embodiment, device 200, e.g., processing unit 250 may be configured to obtain multiple fetal vector cardiograms, e.g., from VCG unit 240, corresponding to different heartbeats of the same fetus from said multiple fetal ECG signals, and to average the multiple fetal VCGs to obtain an averaged fetal VCG. The averaged fetal VCG may then be used as the input to the machine learning classifier. An advantage of averaging after computing VCGs rather than at the stage of fECGs is that signal improvement in the VCG contribute to the quality of the averaged VCG

In an embodiment, device 200, e.g., unit 250 may be configured to determine fetal movement from at least the multiple ECG signals during a measurement period in which the multiple ECG signals are received, and to rotate the multiple fetal VCGs with respect to each other to compensate for the fetal movement before the averaging. For example, one may detect fetal rotation during the measurement period by computing the electric axis for each of the VCGs and rotating the VCGs so that the axes align. Compensating for fetal movement improves the accuracy that can be obtained from averaging VCGs.

When averaging multiple VCGs, one uses the ECGs for multiple heartbeats. However, the fetus may have moved during these heartbeats and therefore may have taken on a different position or orientation towards the abdominal electrodes. Because of these different positions, the ECG changes shape. If one were to average signals when there is movement without correcting for said movement, the signal quality would reduce. In the VCG, correction for movement can be done first so that this problem does not occur.

Instead of aligning axes, one may calculate the rotation between successive VCGs to obtain a more robust result. Moreover, if one were to use the electric axis, only one direction is known and alignment is not yet unambiguously determined.

For example, in an embodiment multiple fetal ECG signals are obtained for multiple fetal heartbeats. Using this information multiple VCGs may be computed corresponding to the multiple heartbeats. All or part of the multiple VCGs may be averaged, preferably after alignment, to obtain one or more low-noise averaged VCGs.

In an embodiment, device 200, e.g., processing unit 250 may normalize the VCG before using it at the classification input, typically after other processing steps such as alignment and/or averaging are done. For example, normalizing may comprise translating the fetal VCG to standardize the mean, and/or scaling the fetal VCG to standardize the standard deviation before providing the fetal VCG as the input. For example, one may normalize a VCG so that the mean of a VCG is made equal to zero with a variance of 1. In an embodiment, truncating or padding of the VCG may be done after the normalization.

It was found that some machine learning classifiers, in particular neural networks, perform poorly on inputs with different amplitudes. Although amplitude information of the fetal ECG/VCG could be valuable in diagnosing CHD, in an embodiment this information is ignored by enforcing normalization before the neural network input. This has two advantages:

- As said, neural networks cannot deal very well with variations in input amplitudes
- Variations in amplitude could also be caused by a fetal heart lying closer or further away from the electrodes, e.g. because of fetal orientation or because of less/more fat in the maternal abdomen. It is thus avoided that amplitude is misinterpreted as caused by CHD while it can be due to higher/lower BMI of the mother. Typically, the same type of normalization is used during training of the classifier as during its use.

Classifier device 200 may comprise a storage 220 for storing trained parameters for a machine learning classifier 260 for classifying a fetal VCG as normal or as abnormal. For example, a fetal VCG obtained by VCG unit 240 or processing unit 250 may be provided as an input to the machine learning classifier configured with the trained parameters. As a result a classification is obtained from the machine learning classifier as an output.

For example, in an embodiment, the machine learning classifier 260 will receive the 3x1024 VCG array, e.g., as obtained through averaging, normalization, and/or truncating / zero-padding.

In an embodiment, the machine learning classifier may comprise a neural network, in particular a deep neural network. For example, the neural network may comprise one or more, two or more, etc., convolutional layers. For example, the neural network may comprise one or more, two or more, etc., fully connected layers. For example, the neural network may comprise one or more, two or more, etc., drop-out layers. For example, in an embodiment, the neural network comprises at least two convolutional layers, at least one drop out layer and at least one fully connected layer. Another type of machine learning classifier is for example, a support vector machine, etc. In an embodiment, drop-out is only used during training, not during operational use when the network is deployed.

The trained parameters for the machine learning classifier, e.g., for the neural network, may have been obtained from a method for training a machine learning classifier for classifying a fetal vector cardiogram as normal or as abnormal. Such a method may comprise training the machine learning classifier on a training set of fetal vector cardiograms. **Figure 3** schematically shows an example of an embodiment of a device 300 for training a machine learning classifier for classifying a fetal vector cardiogram,

Training device 300 comprises a storage for storing a training data 310, a storage 320 for storing parameters for a machine learning classifier, and a machine learning unit 360. For example, machine learning unit 360 may iterate over the training data in storage 310 and iteratively modify the parameters stored in storage 320 so that the classification behavior of a machine learning unit programmed with said parameters better approximates the classification in the training data. For example, the learning algorithm may be reinforcement learning. Various known alternatives to reinforcement learning may be used, e.g., simulated annealing. In an embodiment, the machine learning classifier is trained on a training set of fetal vector cardiograms, wherein the training set comprises fetal vector cardiograms corresponding to fetuses diagnosed with a Congenital Heart Disease (CHD), and fetal vector cardiograms corresponding to fetuses not-diagnosed with a heart condition.

**Figure 4** schematically shows examples of embodiments of training data. For example, training data 410 comprises multiple pairs of a vector cardiogram 412 and a corresponding classification 419. For example, the vector cardiogram 412 may be obtained from an apparatus that is the same or similar to the one used in use of device 200. For example, the vector cardiogram 412 may be obtained by a device that comprises the combination of a signal input 210 for multiple electrodes 211, a fetal ECG unit 230, a vector cardiogram unit 240, and optionally a processing unit 250. The fetal VCG may be obtained at a certain point in gestation, say at or about 20 weeks. The classification may be obtained by screening the born child for, say, CHD. The classification may be a binary classification, e.g., a single bit that indicates that the child had some form of CHD. The classification may be a binary classification that indicates if the child merits further screening, even if it does not quite reach the level of a CHD. The level of sensitivity of device 200 may be arranged by appropriately setting the sensitivity in the training data. In an embodiment, classification 419 may include additional information about the CHD. For example, the classifier may be trained to also classify into a type of CHD and/or a severity of CHD. For example, this may be done by training additional network layers on top of a base network, known as a head. A different head may be used for different classifications. While training for a specific classification the corresponding head is used but the same base network. Instead of multiple heads, one may also use a single head with multiple outputs, etc.

Even if such information in use is not used, e.g., any abnormal classification may in practice be used to select a child for further screening, such additional information during training is believed to improve the accuracy of the machine learning classifier. There may be many pairs 412, 419, e.g., at least a hundred, at least 500, etc.

In an embodiment, device 300 is provided with a set of multiple ECG signals and corresponding classification. In that case, device 300 may perform the same processing, e.g., as implemented in units 230, 240 and/or 250 before using the input data for training the classifier.

Returning to figure 3. An important, but optional, addition to training device 300 is a training data processing unit 350. Processing unit 350 may modify the training data before it is used in training.

In an embodiment, training data processing unit 350 is configured to apply a random rotation to the fetal VCG, e.g., to VCG 412, while keeping the corresponding classification, e.g., classification 419 constant. Augmenting the training set by randomly rotating fetal vector cardiograms before training has the advantage that it prevents the machine learning classifier from associating the classification with fetal orientation. Although it is not known that an association between fetal orientation and chance of CHD exists, this relationship can be avoided during training in this manner. At present this association, if any, is poorly understood. Importantly, there may be other factors that also influence fetal orientation. Moreover, fetal orientation is not constant, regardless of whether the fetus has CHD or not. Accordingly, the inventor found that it would be desirable to have a machine classifier that somehow left the fetal orientation out of its deliberations. This aim is achieved by randomly rotating the VCG during training, so that the machine classifier has no opportunity to associate fetal orientation, e.g., in the form of the orientation of the electrical axis of the VCG, with CHD. As a result a machine learning classifier is obtained that will be less likely to change its classification if the fetal orientation of the fetus changes.

In an embodiment, device 300, e.g., unit 350, is configured for augmenting the training set by adding noise to the training data. Fetal ECG measurements often have a low signal-to-noise ratio, thus by adding noise during the training the classifier is trained to suppress noise. For example, the noise may be Gaussian noise.

Interestingly, two problems which at first glance are similar may advantageously be addressed differently. It is desired that the network does not learn to associate CHD with either fetal orientation with respect to the mother nor with the amplitude of the ECG signal. Although there may be some correlation with CHD both variables also depend on coincidental circumstances, e.g., the current orientation that the fetal happens to be in at the moment of measurement with respect to the electrodes or mother's BMI. To address the first problem, the classifier is trained on VCGs that have been randomly rotated. To address the second problem, the classifier is trained and used on VCGs that have been normalized, e.g., to have a zero mean and standard deviation of 1. The inventor found that these features better address the respective problems, due to, in part, that the neural network does not show a good distinguishing response based on amplitude.

In an embodiment, device 200 and 300 may be configured so that the machine learning classifier receives multiple VCG of the same fetus, e.g., corresponding to sequential heartbeats. For example, the machine learning classifier may be configured to receive multiple fetal vector cardiograms corresponding to different heartbeats of the same fetus as part of the same input. For example, the input may be multiple times 3 times 1024 samples corresponding to multiple VCGs. If it is not possible to use subsequent heartbeats for the VCGs as input, one may use multiple sets of VCG each of which is averaged to produce an averaged VCG as input for the classifier. For example, the average of the VCGs corresponding to a subsequent number of heartbeats may be a first input, and the average of the VCGs corresponding to a further subsequent number of heartbeats may be a second input, etc. When offering multiple VCG the need for averaging is less prominent as the classifier has better opportunity to remove noise itself, e.g., as part of its convolutional layers. In an embodiment, the classifier is provided with averaged VCGs which are obtained by interleaving the data. For example, a first average may be obtained from averaging even heartbeats, and a second average from averaging odd heartbeats; the same may be done with three or more averaged VCGs, etc.

Using multiple VCG as the input for the classifier has advantages. As the device sees multiple VCGs and thus has a better view of the operation of the heart and thus a better chance of detecting a CHD. In particular, some types of CHD, e.g., fetal arrhythmia can be seen only or predominantly from multiple VCGs rather than one VCG.

For example, figure 4 shows an example of training data 420. Example 420 comprises fetal VCGs 422-424. For example, VCGs may correspond to two subsequent heartbeats, or more, or three or more, etc. Training data example 420 further comprises a classification 429. For example, classification 429 may be a binary classification in normal/abnormal, etc., but may also be non-binary classification. For example, classification 429 may comprise a CHD type, e.g., fetal arrhythmia, coarctation of the aorta, etc. For example, a deep neural network may be trained with multiple neural network heads, each combination of a different head and the same neural network is trained for a particular CHD type. If desired a final head may be trained to classify any CHD type; for example after training for specific CHDs is complete.

In an embodiment, device 200, e.g., may comprise multiple classifiers. For example, a first classifier may be configured to receive as input a single VCG, possibly averaged over multiple heartbeats. A second classifier may be configured to receive as input multiple VCGs, possibly not averaged over multiple heartbeats or averaged over fewer or interleaved heartbeats. The first classifier has the advantage of receiving a higher-quality, lower noise VCG, which is better for detecting congenital heart defects that show in each VCG, in the same or approximately the same manner. The second classifier receives lower-quality, higher noise VCGs but has the advantage that it can suppress noise itself, and has the opportunity to see defects that only show up by comparing VCGs of multiple heartbeats.

Returning to figure 2a. Device 200 may further comprise an output unit 270. For example, output unit 270 may be configured to communicate the classification to an operator of the device. For example, output unit 270 may comprise a display or may be connectable to a display. For example, the display may show the output of classifier 260, etc. Additional output may be a confidence indicator that indicates how strong the classifier found indications for CHD. Interestingly, this application may use drop-outs even during deployment.

A confidence estimator may also be implemented by quantifying the quality of the fetal ECG signal and using categorical confidence estimators. For example, the quality of the fetal ECG signal could be described on a 1-5 scale, 5 being the highest quality, 1 being the lowest quality. Low-quality fetal ECG signal would lead to less confidence in the correctness of the CHD detection, so the confidence estimator could be described on the same 1-5 scale, 5 being the highest confidence and 1 the lowest confidence. Instead of a categorical confidence estimator, a continuous estimator may be used, e.g., a confidence indicator on a continuous scale. In case it is difficult to calculate a confidence indicator on a continuous scale with sufficient reliability, it may be easier to assign a discrete value to the confidence indicator.

Alternatively or in addition, one may provide the neural network with more than one fetal vector cardiogram for the same patient and assess the consistence in the CHD detection as a confidence estimator. For example, when all VCGs are classified as originating from CHD the confidence is high, when a few VCGs are classified as CHD while a few others are classified as normal, the confidence is low. Confidence may be estimated from multiple sources, e.g., by assessing the quality of one or more fetal vector cardiograms, or by assessing the consistence in the CHD detection for multiple fetal vector cardiograms, a final confidence value may be computed therefrom, e.g., by taking an average confidence or a minimum of the confidences. For example, consistence may be estimated by computing a variance, e.g., a standard variance, of multiple neural network outputs. A low variance corresponds to a high consistency, and thus to a high confidence.

For example, in practice, during an ultrasound screening a trained sonographer may look for indications of CHD in the image data. In addition, multiple ECG recordings may be made. Using device 200 or 201, the ECG may be automatically classified. For example, if the device finds evidence of CHD, the trained operator may be reminded to reconsider the image data to see if he or she may have missed something, e.g., due to an oversight. Alternatively, the output of the device may be directly used to select patients for further screening.

In an embodiment, device 200 may be configured to provide multiple fetal vector cardiograms corresponding to different heartbeats of the same fetus as an input to the machine learning classifier to obtain multiple classifications. Output unit 270 may be configured to communicate a classification to an operator of the device if at least a threshold number of the multiple classifications indicate said classification. For example, a warning may be generated if at least one of the classifications is abnormal, e.g., indicates possible CHD.

**Figure 2b** schematically shows an example of an embodiment of a device 201 for processing a fetal electrocardiogram. Device 200 is the same or similar as device 201 but uses an additional input for improved detection of CHD. Compared to device 200, device 201 has a further input: orientation input 280. Input 280 may be connectable to an orientation input device 281. For example, orientation input device 281 may be an app running on a smart phone. Using the orientation input device 281, medical personal can indicate the orientation of the fetus during the measurement period, e.g., with respect to the mother. Multiple inputs may be provided, in case the fetus moves during measurement. For example, device 200, e.g., input 280 may be configured to receive fetal orientation. Device 200, e.g., processing unit 250 may be configured to calculate the orientation of the electrical heart axis with respect to the fetal orientation. The trained parameters 221 have been trained to take the orientation into account, possibly in the form of an additional input.

An advantage of having the orientation of the electrical heart axis as an input is that a CHD that cause deviations in the heart axis may be more easily detected. For example, the orientation may be an additional 3 element input to the network. For example, the orientation may be represented as a vector. For example, the vector may be normalized to have an Euclidean length of 1. Other input representation may be used, e.g., polar coordinates, etc. Providing the orientation of the electrical heart axis to the machine learning classifier may improve detections of some types of CHD. Other normalization may normalize amplitude or energy. In an embodiment, a 3x3 rotation matrix is used. A rotation matrix R has a number of specific properties:

- Determinant is equal to 1
- R * R' = I (I identity matrix)

The information in a 3x3 rotation matrix may be summarized in a 3x1 vector in which the elements in that vector describe the rotations in different directions. In the above formula R' denotes the transpose of R.

As an alternative to an additional orientation input, the fetal VCG could also be rotated to correct for the fetal orientation, e.g., by normalizing the orientation. Then the orientation itself doesn't need to be provided to the machine learning classifier. For example, such a normalization could be performed in the unit 250. The same type of normalization and/or orientation input is provided in training as well as during use of the classifier.

Orientation can be obtained from, e.g., palpation, e.g., feeling from the outside how the fetus lies, or from imaging techniques, typically ultrasound, but MRI would work as well. Once the orientation of the fetus, e.g., the orientation with respect to the mother has been determined, the orientation may be inputted to device 200. For example, if the orientation is obtained manually, e.g., using imaging or palpitation, then the orientation may be inputted. For example, the orientation may be inputted on device 200, e.g., using input means such as buttons, sliders, a mouse and the like. An external input device such as input device 281 may also be used. If electronic imaging is used, the fetus orientation may be derived automatically as well, possibly on a continuous basis. This would improve signal accuracy of the fetal vector cardiogram.

Figure 4 shows training data example 430. Training data example 430 comprises a vector cardiogram 432, an orientation 434, and a classification 439. For example, the orientation may be the orientation of the electrical axis of the fetal heart with respect to the fetus.

In an embodiment, the training data provides multiple VCGs together with multiple associated orientations.

Some CHD cause deviations in the orientation of the electrical heart axis and to a smaller, or even none, extent to the shape of the VCG. In an embodiment, information on the fetal orientation, e.g., obtained with ultrasound, is used to define the fetal VCG within the fetal frame of reference. One could consider this as a virtual fetal VCG, as if recorded with electrodes directly on the fetal body, e.g., in a configuration similar to that used in adult electrocardiography. An advantage of this approach is that it combines the VCG and information on possible deviations in the orientation of the electrical heart axis. A possible disadvantage of embedding the orientation information in the VCG is that it prevents data augmentation via rotations, as it would lose the information on the orientation again.

In an embodiment, a VCG is combined with embedded orientation information to detect CHD. This may come at the expense of some additional work during measurements, e.g., to determine and input the fetal orientation. Such an approach is preferably trained with an amount of training data that is large enough so that different fetal orientations are sufficiently available in both cases. For example, one may verify that the network does not associate fetal position with CHD and that the network converges. In such a rich dataset, augmentation may no longer be needed.

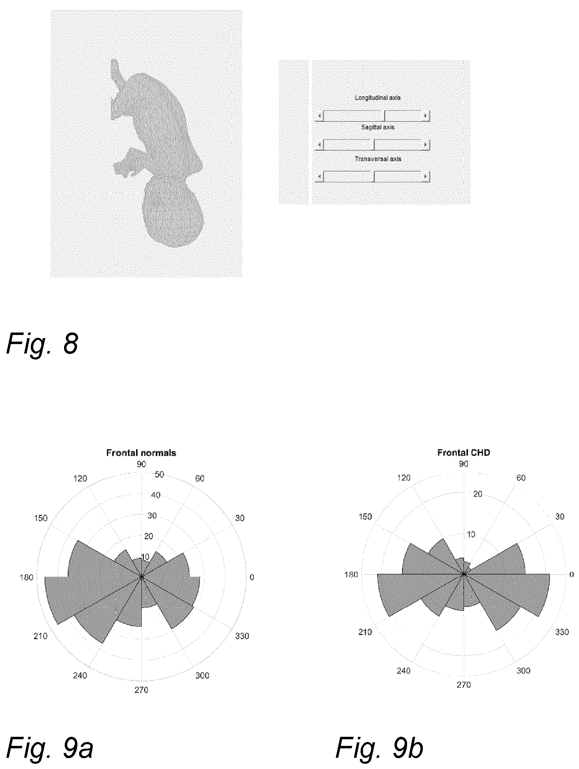

**Figure 8** schematically shows an example of an embodiment of a device for inputting fetal orientation. Shown on the device is a picture of a fetus. The picture represents a generic picture of a fetus. Using a number of sliders, the orientation of the fetus in the picture can be changed until it matches the orientation of the fetus in the womb. At that point, the device may supply the orientation to device 201. For example, an operator of device 281 may press a submit button.

In an embodiment, the three sliders shown in figure 8 allow the child to rotate along the longitudinal axis (head-foot), transverse axis (left-right side) and frontal axis (belly-back). When, say, a doctor rotates the image, a 3x3 rotation matrix may be calculated and/or updated in the background that describes the rotation of the image relative to the initial position. In the initial position, the fetus might sit upright and look at the user.

**Figure 9a** schematically shows an example of a histogram of fetal orientation for fetuses without CHD. **Figure 9b** schematically shows an example of a histogram of fetal orientation for fetuses with CHD,

The histograms show the orientation of the heart axis for a number of children, both in a healthy group and a group with CHD. The latter group is very heterogeneous in the sense that the children have different heart conditions: in some no deviating heart axis is expected, in others a deviation is expected. In an embodiment, this may be further differentiated per heart defect, as further data becomes available. For the healthy children it can be seen that the majority has a heart-axis that is in the third quarter below. From the patient's perspective, this is at the bottom right.

After birth, the left ventricle usually has to deliver the most labor: it pushes the blood into the aorta to supply the whole body with oxygen. As a result, the left ventricle has the most muscle mass and dominates it electrically. A fetus has a different circulation though, including a hole between left and right half of the heart (the foramen ovale). Because of this different circulation, the right ventricle produces an estimated 60% of the labor versus 40% for the left of the fetus. As a result, one also expects a domination of the right ventricle, and accordingly an electric heart axis to the bottom right. Note that, ventricles are at the bottom of the heart, atriums are at the top. Figures 9a and 9b thus show the efficacy of providing fetal orientation to the classifier.

The described technology - fetal ECG-based detection of congenital heart disease (CHD) - might be employed as a stand-alone solution, but further improvement in the performance of CHD detection in clinical practice is expected when fetal ECG and ultrasound analysis are employed together. Some CHD affect the special system in the heart that conducts electrical impulses to make sure the cardiac muscles contract in the right order and at the right moment. Abnormalities in this conduction system can cause suboptimal cardiac functioning. For these CHD, ECG-based detection is best suited because the ECG reveals information on the electrical activity of the heart. Especially at relative early gestational ages, the heart might not have adapted to these electrical abnormalities in the extent that it has affected the anatomy of the heart in such a way that it can be assessed by ultrasound examination. Other CHD can cause anatomical defects and might affect the electrical system to a lesser extent, or even not at all. These CHD are best detected via ultrasound.

A system that combines FECG and ultrasound has the additional advantage that the ultrasound can be used to determine the fetal orientation. This information allows the detection of abnormalities in the orientation of the electrical heart axis, which can further boost detection rates of CHD, as described already in the current patent application.

In an embodiment, ultrasound and fetal ECG may be combined in a single device and/or method. For example, ultrasound and fetal ECG may be combined in a single device with automated analysis for CHD in at least one or both of these modalities. It could be that for the ultrasound the input of the sonographer is required and that for instance a confidence estimator of the final decision depends on the level of agreement between sonographer and neural network. In an embodiment, the analysis of the ultrasound is automated; this may be done using a neural network, e.g., that is trained to detect CHD in the fetal heart from the ultrasound images.

It is not necessary to detect CHD directly from the ultrasound images. Although possible, such detection is hard because of a challenging resolution of the images, while the fetal heart is small and the ultrasound probe cannot be placed close to this heart. Interestingly, the fetal orientation may be determined from the ultrasound data, e.g., images. The determined fetal orientation may be used as a fetal orientation inputs, as described herein. For example, in an embodiment, two machine learning units, e.g., neural networks may be used. A first network may receive ultrasound data, and optionally information on the orientation of the ultrasound probe, and may compute a fetal orientation therefrom. A second network may receive fetal ECG data, e.g., a fetal VCG and the orientation determined by the first network and classify the VCG data, e.g., detect CHD.

From orientation information of the fetus and the fetal VCG, one can calculate the orientation of the electrical heart axis. The fetal orientation can also be used to rotate the fetal VCG to normalize it for the fetal orientation. In other words, an embodiment may obtain a fetal VCG as if it were recorded by electrodes on the fetal body. This way, information on the fetal orientation and/or electrical heart axis would be embedded in the VCG. Automated detection of the fetal orientation based on ultrasound has the advantage that orientation may be obtained faster and more accurately than using human estimation only.

In an embodiment, ultrasound resolution and/or quality in 3D and/or 4D applications may be improved by combining ultrasound clips with information on cardiac intervals obtained from the fetal ECG/VCG, using a method for automated detection of the cardiac intervals, e.g. by a neural network or by more conventional feature detection methods. For example, in 3D ultrasound, the goal may be to make a short movie of a single cardiac contraction in 3D. However, ultrasound scanners cannot scan the full 3D space fast enough to track all motion of the cardiac walls. As a solution, they scan the heart during several heartbeats and combine all these data to create a 3D movie of an artificial heartbeat, e.g., a single heartbeat that is re-created by combining multiple small pieces of successive heartbeats. This combination is based on spatiotemporal image correlation techniques (STIC), but these are inaccurate and cause blurring of the resulting movie.

Automated analysis of the cardiac functioning, by e.g. using speckle tracking techniques is hence complicated because of the poor quality of the images/video.

With simultaneously acquired fetal ECG intervals, an accurate timing mechanism is available. Specific intervals in the ECG can be related to movement of the cardiac walls. With such an external source of information, images of successive heartbeats or parts of heartbeats can be synchronized more accurately than by STIC. This will reduce the blurring so that images are better interpretable. Note that, an embodiment may be configured for automated detection of ECG intervals and using these intervals for ultrasound image enhancement. For example, the fetal ECG may be used as trigger to synchronize ultrasound.

For example, an embodiment of a device for processing a fetal ultrasound images may comprise

- a signal input for receiving multiple ultrasound images from an ultrasound probe in multiple positions and/or from multiple time-points, said ultrasound probe being configured to be arranged on the abdomen of a pregnant woman,
- a storage for storing trained parameters for a machine learning classifier. Said machine learning classifier being configured to receive ultrasound data, e.g., one or more or all of the multiple ultrasound images.
- a processor configured to

  - obtain the ultrasound data from the multiple ultrasound images,
  - provide the ultrasound data as an input to the machine learning classifier configured with the trained parameters.

There are several options. For example, the ultrasound data may be one or more ultrasound images. For example, the ultrasound data may represent an artificial heartbeat, e.g., reconstructed as indicated above from the multiple ultrasound images. The machine learning classifier for ultrasound may provide a classification, e.g., as the machine learning classifier for ECG data does, or it may provide a fetal orientation. In an embodiment, the machine learning classifier receives both fetal ECG and ultrasound data, e.g., a reconstructed fetal heartbeat. For example, the ultrasound data show the contraction of the fetal cardiac walls, e.g., in a series of multiple images, possibly reconstructed images. A machine learning classifier that is trained on ultrasound data, possibly in addition to ECG data can detect different CHD cases than one that is only trained on ECG data. Ultrasound training data may be put together and/or augmented in the same manner as disclosed herein for ECG data. If two machine learning classifiers are used, e.g., one trained for ultrasound data and one for fetal ECG data, the two outputs may be communicated, and possibly combined.

In the various embodiments of the classification devices, e.g., device 200 and 201, and the training devices, e.g., device 300, communication interfaces may be selected from various alternatives. For example, communication interface may be a network interface to a local or wide area network, e.g., the Internet, a storage interface to an internal or external data storage, a keyboard, an application interface (API), etc.

The classification and training devices may have a user interface, which may include well-known elements such as one or more buttons, a keyboard, display, touch screen, etc. The user interface may be arranged for accommodating user interaction, e.g., for performing a classification or for performing a training.

The storages, e.g., storage 220, 310 and 320 may be implemented as an electronic memory, say a flash memory, or magnetic memory, say hard disk or the like. A storage may comprise multiple discrete memories together making up the storage.

Typically, a classification and training device, e.g., devices 200, 201, 300 each comprise a microprocessor which executes appropriate software stored at the device; for example, that software may have been downloaded and/or stored in a corresponding memory, e.g., a volatile memory such as RAM or a non-volatile memory such as Flash. Alternatively, the devices may, in whole or in part, be implemented in programmable logic, e.g., as field-programmable gate array (FPGA). The classification and training devices may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc.

In an embodiment, a classification comprises a fetal ECG circuit, a vector cardiogram circuit, a processing circuit and a machine learning classifier circuit. In an embodiment, a training device comprises a training data processing circuit, a machine learning circuit. The circuits implement the corresponding units described herein. The circuits may be a processor circuit and storage circuit, the processor circuit executing instructions represented electronically in the storage circuits.

A processor circuit may be implemented in a distributed fashion, e.g., as multiple sub-processor circuits. A storage may be distributed over multiple distributed sub-storages. Part or all of the memory may be an electronic memory, magnetic memory, etc. For example, the storage may have volatile and a non-volatile part. Part of the storage may be read-only. The circuits may also be, FPGA, ASIC or the like.

Below a number of additional non-limiting details and/or additional non-limiting embodiments are described. A study towards the use of fetal electrocardiography for detection of CHD around 20 weeks of gestation in a cohort of almost 500 pregnancies was performed. Measurements were performed directly before or after the 20 week fetal anomaly scan. This ultrasound scan was performed by a certified and experienced sonographer. Patients carried a singleton fetus with a gestational age between 18 and 24 weeks.

Patients with an uneventful pregnancy were assigned to the normal cohort; patients that were diagnosed with the CHD during the 20-week ultrasound scan, later in pregnancy, or postnatally, were assigned to the CHD cohort. In total, 496 patients were measured of which 4 were lost to follow-up.

Of the total of 492 patients, 347 were assigned to the normal cohort and 145 to the CHD cohort. There are various types of CHD, for example: TGA: transposition of great arteries; ToF: tetralogy of Fallor; VSD: ventricular septal defect; AVSD: atrioventricular septal defect, etc.

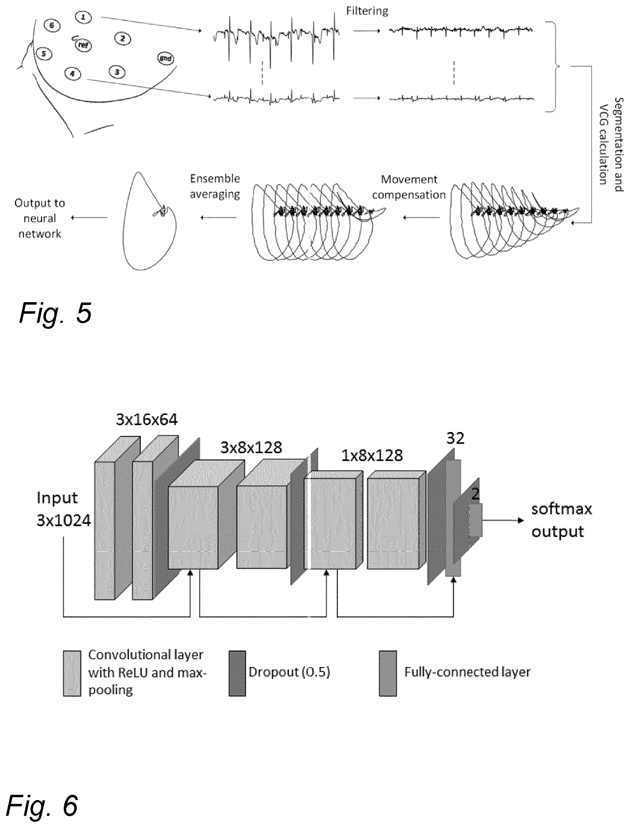

The fetal ECG was recorded with adhesive Ag/AgCl electrodes on the abdomen of the pregnant women while she lay in a semi-recumbent position. In total, eight electrodes were placed on the abdomen in a fixed configuration (see Figure 5) that provide six bipolar channels of electrophysiological measurements; two electrodes served as common reference and ground. The duration of the registration was 30 minutes.

**Figure 5** schematically shows an example of an embodiment of a method of processing a fetal electrocardiogram, and in particular data acquisition and processing. The abdominal electrodes provide in total six electrophysiological signals, of which only two are displayed in the upper-center panel of figure 5, that mainly contain maternal ECG. After filtering, the fetal ECG becomes visible and is segmented into individual heartbeats after which the fetal VCG can be calculated. In the bottom-right panel, fetal VCGs for multiple consecutive heartbeats are displayed during an episode of fetal movement; the VCGs are rotated with respect to one another. In the bottom-center panel, the VCGs have been compensated for fetal movement, after which they are averaged to yield a high-quality fetal VCG (bottom-left panel) that serves as input to the neural network.

The electrophysiological signals were digitized and stored at 500 Hz sampling frequency by a prototype fetal monitoring system. After digitization, the acquired signals were processed by PC-based signal processing techniques as previously described by Vullings et al.[28,29], Warmerdam et al.[30,31], and Fotiadou et al.[32] to remove interferences such as the maternal ECG, powerline, and electromyographic signals from within the maternal body (see also Figure 5) and identify fetal QRS complexes. This signal processing was performed for each of the six channels of fetal ECG data.

Subsequently, the fetal ECG signals were segmented in such a way that every segment contained exactly one heartbeat and that the segments were synchronized on the position of the R-peak. When insufficient fetal QRS complexes were detected, the recording was excluded from further analysis due to low signal quality. In an embodiment, an average of at least 25 detected fetal QRS complexes per minute as threshold for signal quality was used.

To further enhance the fetal ECG signals, multiple ECG complexes from consecutive heartbeats are averaged to suppress temporally uncorrelated noise. However, the electrodes are positioned on the maternal abdomen and the fetus is free to move throughout the uterus, causing it to move with respect to the abdominal electrodes. This can cause each specific fetal ECG channel, e.g., lead, to change with fetal movement. To draw an analogy with adult cardiology; fetal movement could be regarded as changing the electrode positions during an adult ECG examination. By averaging ECG complexes of various heartbeats during which the fetus moved, the fetal ECG will be blurred, exactly opposite from the targeted ECG enhancement.

Fortunately, fetal movement can be compensated via a two-step process. In the first step, the fetal vector cardiogram (VCG) is calculated[28], which represents a three-dimensional representation of the fetal ECG. This fetal VCG rotates in three-dimensional space with movements of the fetal trunk and these rotations can be tracked and corrected for via a mathematical method presented in [33]. As a result, for every heartbeat a fetal VCG is obtained that is spatially aligned with the first VCG in the recording. Now that fetal movement has been compensated for, one can calculate the ensemble average of the VCGs to obtain a single high-quality fetal VCG, as illustrated in the bottom left panel of Figure 5. Here, one may use the average over 250 consecutive heartbeats.

Depending on the average fetal heart rate during the recording and the exact recording length, per fetus more than one VCG could be determined; on average, per fetus 12 VCGs are determined. This is possible even if each VCG is obtained as the average of multiple unique heartbeats. For example, 250 fetal VCGs or 250 fetal ECGs may be averaged to obtain a high quality VCG and still multiple of such high-quality VCG may be obtained.

To determine which fetal VCG morphology corresponds to a healthy fetal heart and which VCG can be associated with CHD, a deep neural network may be employed. The network architecture consists of 6 convolutional layers with residual connections, ReLU (rectified linear unit) activation, and max-pooling. These convolutional layers are followed by two fully-connected layers and a softmax function. An Adam optimizer may be employed and used dropout for regularization. Further details of the network are provided in Figure 6.

Figure 6 schematically shows an example of an embodiment of neural network for classifying a fetal VCG. Figure 6 shows a possible architecture and details of the neural network. Each convolutional block may be followed by a ReLU activation and max-pooling. Batch normalization was applied to expedite the training (for clarity not depicted in figure 6). Inputs in these embodiments are resampled to 2 kHz and truncated to 1024 samples (512 ms). Other sampling rates, or not using resampling at all, is also possible.

Prior to training the neural network, 100 patients were selected for testing the performance of the network. These patients were selected to ensure there were various CHD cases, as well as controls, in this test population. The data of the remaining 392 patients was used for training the network; a randomly selected 90% of these patients was used in training, the other 10% for validation to prevent overfitting.

Since the fetal orientation is unknown and could be virtually any position, the training data may be augmented, e.g., fourfold, by randomly rotating every fetal VCG. By randomly rotating the fetal VCGs, it is prevented that the neural network wrongfully learns that the probability of having CHD is related to fetal orientation. At the same time, it is ensured that the performance of CHD detection is independent on the fetal orientation. Another fourfold augmentation was performed by adding Gaussian-distributed noise to the training data. As reference data, one may use binary classes (i.e. 0 meaning a participant from the normal cohort and 1 meaning a participant from the CHD cohort).

After the neural network has been trained, it is evaluated by providing it with the - until then - unseen data of the test population. For each fetal VCG in the test set, the network classifies it as normal or as CHD. As mentioned before, per fetus typically about 12 VCGs are determined. When at least a specific fraction of these VCGs is classified as CHD by the network, the fetus is considered to be classified in the CHD group. We evaluated several fractions between 0 and 1, allowing for the compilation of a receiver-operating-characteristic (ROC) curve. The scenario where the fetus was considered to be classified in the CHD group when at least one of the VCGs was considered abnormal by the neural network was also evaluated.

As outcome measures, one may use sensitivity and specificity, accuracy and F1-score of the neural network in detecting CHD in the test set. These measures are defined according to:

$$Sensitivity = \frac{TP}{TP + FN}, Specificity = \frac{TN}{TN + FP},$$

$$Accuracy = \frac{TP + TN}{TP + FP + TN + FN}, F1 = \frac{2TP}{2TP + FP + FN}.$$

Here, TP is a true positive (i.e. correct CHD detection), TN is true negative, FP is false positive, and FN is false negative.

From the 492 measurements that were performed, 480 had sufficient quality for further analysis. From these 480 usable recordings, 343 are from the normal cohort and 137 are from the CHD cohort. Prior to analysis, 100 patients were selected for the test set. Of these 100 recordings, 99 (50 from the normal cohort, 49 from the CHD cohort) were usable. As a result, 381 recordings (293 normal, 88 CHD) could be used for training the neural network.

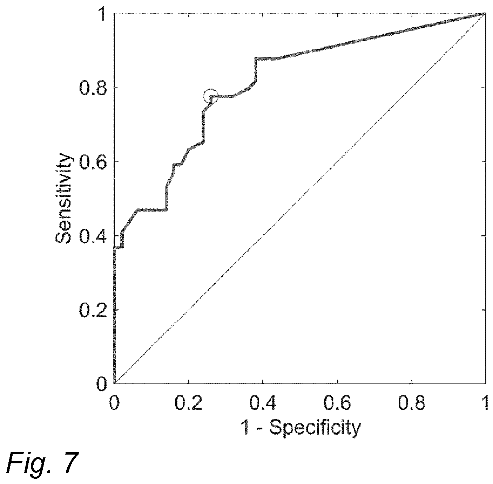

For each fraction of VCGs per patient that was classified as CHD by the neural network, Sensitivity and Specificity were computed. These results are depicted in **Figure 7** as an ROC curve. For a fraction of at least 11.5% of VCGs per patient classified as abnormal, our method obtained the highest combination of Sensitivity and Specificity: 77.6% and 74.0%, respectively. Accuracy was 75.8% and F1-score 76%.

**Figure 7** schematically shows an example of sensitivity for an embodiment of the device for processing a fetal electrocardiogram, in particular the ROC curve of the detection of CHD. Here, sensitivity and specificity are determined as a function of the fractions of VCGs that were labelled as abnormal for each patient for that patient to be classified as from the CHD cohort. The circle represents an optimal balance of sensitivity and specificity, specified as the point closest to the upper-left corner.

When the fetus was considered to be classified as from the CHD cohort when at least one of its VCGs was classified as abnormal, the Sensitivity and Specificity of our method were 87.8% and 56.0%, respectively. Accuracy and F1-score were 71.7% and 75.4%, respectively.

Considering the scenario with fraction of 11.5% as described above, 24 recordings were classified wrongly by our method, of which 13 were normal. The 11 CHD cases that were classified wrongly had conditions TGA (2 cases of which one combined with VSD), Taussig-Bing (1 case), ToF (3 cases), right-isomerism combined with AVSD (1 case), VSD (4 cases, one combined with TGA, one combined with coarctation of the aorta, and one combined with truncus arteriosus), and ASVD (1 case). Note that the patient with combined TGA and VSD is mentioned twice; once as TGA case, once as VSD.

Using the above embodiment for prenatal screening for CHD, CHD can be detected around 20 weeks of gestation with a sensitivity and specificity of 77.6% and 74.0%, respectively. This performance is independent of the expertise and experience of the operator, say, a sonographer, that performs the prenatal screening, as is the case with performance of ultrasound-based screening.

In an embodiment, 250 fetal heartbeats were used to provide a high-quality fetal VCG. A high-quality fetal VCG was obtained in 97.6% of the patients. The number of 250 heartbeats may be reduced to a smaller number, e.g., by accepting a lower quality VCG or with additional signal enhancement methods. Note, that the practical downside of a high number of VCGs is relatively small. Considering a typical fetal heartrate of about 140 beats per minute, it takes only 2 minutes to acquire 250 fetal heartbeats. In an embodiment, several fetal VCGs per patient were used, but by reducing the number of these VCGs or by making them partly overlapping, it is expected expect that reliable ECG-based CHD assessment can be done with only 10 minutes of fetal ECG data. It should be mentioned here that these fetal ECG measurements can be performed simultaneously with the ultrasound screening that typically last longer than 10 minutes.

The described method achieved higher detection rates than those reported for standard ultrasound screenings. In one embodiment, ECG complexes of 250 heartbeats were averaged to enhance the quality of the signals. As a result, inter-beat variations in the morphology of the ECG are lost in our method and that embodiment can only detect structural electrical abnormalities. However, with additional/other signal enhancement methods the number of averages may be reduced to the extent where inter-beat variations become visible and the method could also be used to study arrhythmia.

Note that some CHD manifest themselves by alterations in the orientation of the electrical heart axis. For most of the recordings in our dataset, information on the fetal orientation was available and by including this information, one can calculate the orientation of the electrical heart axis[34]. This yield additional information to increase the CHD detection rates.

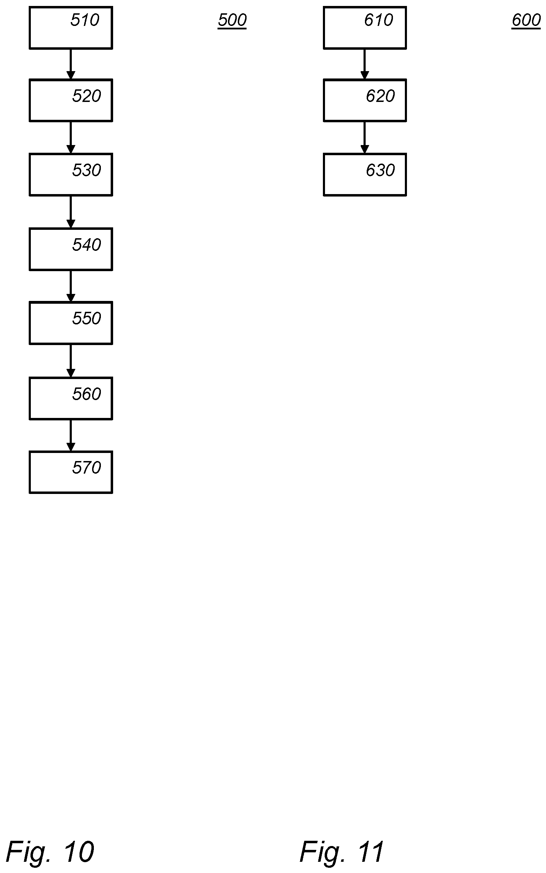

**Figure 10** schematically shows an example of an embodiment of a method 500 for processing a fetal electrocardiogram. The method comprises

- receiving (510) multiple ECG signals from multiple electrodes (211), said electrodes being configured for arranging on the abdomen of a pregnant woman,
- storing (520) trained parameters for a machine learning classifier for classifying a fetal vector cardiogram as normal or as abnormal,
- obtaining (530) at least a partial fetal ECG signal from the multiple ECG signals , and calculating (540) at least one fetal vector cardiogram (VCG) from said multiple fetal ECG signals,
- providing (550) the fetal vector cardiogram as an input to the machine learning classifier configured with the trained parameters, and obtaining (560) a classification from the machine learning classifier,
- communicating (570) the classification to an operator of the device.

**Figure 11** schematically shows an example of an embodiment of a method 600 for training a machine learning classifier for classifying a fetal vector cardiogram. The method comprises

- receiving (610) a first training set of fetal vector cardiograms and a corresponding second training set of classifications,
- augmenting (620) the first training set by randomly rotating fetal vector cardiograms before training to prevent the machine learning classifier from associating the classification with fetal orientation.
- providing (630) the first and second training set to a machine learning algorithm to obtain trained parameters for the machine learning classifier.

Many different ways of executing the method are possible, as will be apparent to a person skilled in the art. For example, the order of the steps can be performed in the shown order, but the order of the steps can be varied or some steps may be executed in parallel. Moreover, in between steps other method steps may be inserted. The inserted steps may represent refinements of the method such as described herein, or may be unrelated to the method. For example, some steps may be executed, at least partially, in parallel. Moreover, a given step may not have finished completely before a next step is started.

Embodiments of the method may be executed using software, which comprises instructions for causing a processor system to perform method 500 and/or 600. Software may only include those steps taken by a particular sub-entity of the system. The software may be stored in a suitable storage medium, such as a hard disk, , a memory device, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the invention also extends to computer programs, particularly computer programs on or in a carrier, adapted for putting the invention into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the means of at least one of the systems and/or products set forth.

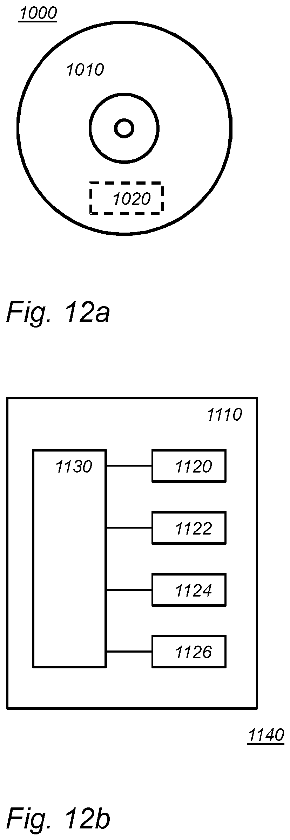

**Figure 12a** shows a computer readable medium 1000 having a writable part 1010 comprising a computer program 1020, the computer program 1020 comprising instructions for causing a processor system to perform a classifying and/or training method, according to an embodiment. The computer program 1020 may be embodied on the computer readable medium 1000 as physical marks or by means of magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said classifying and/or training method.

**Figure 12b** shows in a schematic representation of a processor system 1140 according to an embodiment. The processor system comprises one or more integrated circuits 1110. The architecture of the one or more integrated circuits 1110 is schematically shown in Figure 12b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively. For example, communication element 1126 may be arranged to receive ECG signals from multiple electrodes, either directly or indirectly, or to receive other data, e.g., training data, trained parameters and the like.

For example, in an embodiment, processor system 1140, e.g., the classifying and/or training device may comprise a processor circuit and a memory circuit, the processor being arranged to execute software stored in the memory circuit. For example, the processor circuit may be an Intel Core i7 processor, ARM Cortex-R8, etc. In an embodiment, the processor circuit may be ARM Cortex M0. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

It should be noted that the above-mentioned embodiments illustrate rather than limit the invention, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of" when preceding a list of elements represent a selection of all or of any subset of elements from the list. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

In the claims, references in parentheses refer to reference signs in drawings of exemplifying embodiments or to formulas of embodiments, thus increasing the intelligibility of the claim. These references shall not be construed as limiting the claim.

**Claims**

1. Device (200) for processing a fetal electrocardiogram (fECG) to detect a Congenital Heart Disease (CHD), the device comprising

 - a signal input (210) for receiving multiple ECG signals from multiple electrodes (211), said electrodes being configured for arranging on the abdomen of a pregnant woman,
 - a storage (220) for storing trained parameters for a machine learning classifier for classifying a fetal vector cardiogram as having a CHD or not, wherein the trained parameters have been obtained from a method for training a machine learning classifier for classifying a fetal vector cardiogram as having a CHD or not, the method comprising training the machine learning classifier on a training set of fetal vector cardiograms, wherein the training set comprises fetal vector cardiograms corresponding to fetuses diagnosed with a CHD, and fetal vector cardiograms corresponding to fetuses not-diagnosed with a heart condition,
 - a processor configured to

 - obtain multiple fetal ECG signals from the multiple ECG signals, and calculate at least one fetal vector cardiogram (VCG) from said multiple fetal ECG signals,

- provide the fetal vector cardiogram as an input to the machine learning classifier configured with the trained parameters, and obtain a classification from the machine learning classifier, wherein calculating the fetal VCG is performed on a higher sample-rate, the fetal vector cardiogram being down-sampled to a lower sample-rate before providing as an input to the machine learning classifier,

- communicate the classification to an operator of the device.

2. Device for processing a fECG as in Claim 1, wherein the method for training the machine learning classifier comprises training the machine learning classifier on a training set of fetal vector cardiograms, wherein the training set is augmented by randomly rotating fetal vector cardiograms before training to prevent the machine learning classifier from associating the classification with fetal orientation.

3. Device for processing a fECG as in any one of the preceding claims, wherein the machine learning classifier is a deep neural network.

4. Device for processing a fECG as in any one of the preceding claims, wherein the processor is configured to

- obtain multiple fetal vector cardiograms corresponding to different heartbeats of the same fetus from said multiple fetal ECG signals,
- average the multiple fetal VCGs to obtain an averaged fetal VCG, the averaged fetal VCG being provided as the input to the machine learning classifier.

5. Device for processing a fECG as in Claim 4, wherein the processor is configured to

- determine fetal movement from at least the multiple ECG signals during a measurement period in which the multiple ECG signals are received,
- rotate the multiple fetal VCGs with respect to each other to compensate for the fetal movement before the averaging.

6. Device for processing a fECG as in any one of the preceding claims, wherein the processor is configured to

- detect fetal QRS complexes in the fetal ECG signals,
- reject the received multiple ECG signals if the number of detected fetal QRS complexes is below a threshold.

7. Device for processing a fECG as in any one of the preceding claims, wherein the processor is configured to

- translate the fetal VCG to standardize the mean, and/or scale the fetal VCG to standardize the standard deviation before providing the fetal VCG as the input.

8. Device for processing a fECG as in any one of the preceding claims, wherein the processor is configured to

- provide multiple fetal vector cardiograms corresponding to different heartbeats of the same fetus as an input to the machine learning classifier to obtain multiple classifications,
- communicate a classification to an operator of the device if at least a threshold number of the multiple classifications indicate said classification.

9. Device for processing a fECG as in any one of the preceding claims, wherein the machine learning classifier is configured to receive multiple fetal vector cardiograms corresponding to different heartbeats of the same fetus as part of the same input, the training set comprising fetal vector cardiograms corresponding to fetuses diagnosed with fetal arrhythmia.

10. Device for processing a fECG as in any one of the preceding claims, wherein the processor is configured to

- receive fetal orientation and calculate the orientation of the electrical heart axis with respect to the fetal orientation, and wherein the processor is configured to
- provide the orientation of the electrical heart axis to the machine learning classifier, or
- normalize the orientation by rotating the fetal VCG to correct for the fetal orientation.

11. Device for processing a fECG as in any one of the preceding claims, wherein the method for training the machine learning classifier comprises augmenting the training set by adding noise to the training data.

12. Device for processing a fECG as in any one of the preceding claims, wherein the processor is configured to compute a confidence estimation for the classification.

13. Device for processing a fECG as in any one of the preceding claims, wherein the processor is configured to provide ultrasound data as a further input to the machine learning classifier.

14. A computer readable medium (1000) comprising transitory or non-transitory data (1020) representing instructions to cause a processor system to perform:
a method for processing a fetal electrocardiogram (fECG) to detect a Congenital Heart Disease (CHD), the method comprising

- receiving multiple ECG signals from multiple electrodes, said electrodes being configured for arranging on the abdomen of a pregnant woman,
- storing trained parameters for a machine learning classifier for classifying a fetal vector cardiogram as having a CHD or not, wherein the trained parameters have been obtained from a method for training a machine learning classifier for classifying a fetal vector cardiogram as having a CHD or not, the method comprising training the machine learning classifier on a training set of fetal vector cardiograms, wherein the training set comprises fetal vector cardiograms corresponding to fetuses diagnosed with a CHD, and fetal vector cardiograms corresponding to fetuses not-diagnosed with a heart condition,
- obtaining multiple fetal ECG signals from the multiple ECG signals, and calculating at least one fetal vector cardiogram (VCG) from said multiple fetal ECG signals,
- providing the fetal vector cardiogram as an input to the machine learning classifier configured with the trained parameters, and obtaining a classification from the machine learning classifier, wherein calculating the fetal VCG is performed on a higher sample-rate, the fetal vector cardiogram being down-sampled to a lower sample-rate before providing as an input to the machine learning classifier,
- communicating the classification to an operator of the device.


## Patentansprüche

1. Vorrichtung (200) zum Verarbeiten eines fetalen Elektrokardiogramms (fECG) zum Detektieren einer angeborenen Herzerkrankung (CHD), wobei die Vorrichtung umfasst

- einen Signaleingang (210) zum Empfangen mehrerer ECG-Signale von mehreren Elektroden (211), wobei die Elektroden zum Anordnen am Bauch einer schwangeren Frau konfiguriert sind,
- eine Speichervorrichtung (220) zum Speichern trainierter Parameter für einen Maschinenlernklassifizierer zum Klassifizieren eines fetalen Vektorkardiogramms als eine CHD aufweisend oder nicht, wobei die trainierten Parameter aus einem Verfahren zum Trainieren eines Maschinenlernklassifizierers zum Klassifizieren eines fetalen Vektorkardiogramms als eine CHD aufweisend oder nicht erhalten wurden, wobei das Verfahren Trainieren des Maschinenlernklassifizierers an einem Trainingssatz von fetalen Vektorkardiogrammen umfasst, wobei der Trainingssatz fetale Vektorkardiogramme, die mit CHD diagnostizierten Feten entsprechen, und fetale Vektorkardiogramme umfasst, die nicht mit einer Herzerkrankung diagnostizierten Feten entsprechen,
- einen Prozessor, der konfiguriert ist zum
- Erhalten mehrerer fetaler ECG-Signale aus den mehreren ECG-Signalen, und Berechnen mindestens eines fetalen Vektorkardiogramms (VCG) aus den mehreren fetalen ECG-Signalen,
- Bereitstellen des fetalen Vektorkardiogramms als eine Eingabe an den mit den trainierten Parametern konfigurierten Maschinenlernklassifizierer, und Erhalten einer Klassifizierung von dem Maschinenlernklassifizierer, wobei Berechnen des fetalen VCGs mit einer höheren Abtastrate durchgeführt wird, wobei das fetale Vektorkardiogramm auf eine niedrigere Abtastrate unterabgetastet wird, bevor es dem Maschinenlernklassifizierer als eine Eingabe bereitgestellt wird,
- Kommunizieren der Klassifizierung an einen Bediener der Vorrichtung.

2. Vorrichtung zum Verarbeiten eines fECGs nach Anspruch 1, wobei das Verfahren zum Trainieren des Maschinenlernklassifizierers Trainieren des Maschinenlernklassifizierers an einem Trainingssatz von fetalen Vektorkardiogrammen umfasst, wobei der Trainingssatz durch zufällig rotierende fetale Vektorkardiogramme vor dem Training erweitert wird, um zu verhindern, dass der Maschinenlernklassifizierer die Klassifizierung mit der fetalen Orientierung assoziiert.

3. Vorrichtung zum Verarbeiten eines fECGs nach einem der vorhergehenden Ansprüche, wobei der Maschinenlernklassifizierer ein tiefes neuronales Netzwerk ist.

4. Vorrichtung zum Verarbeiten eines fECGs nach einem der vorhergehenden Ansprüche, wobei der Prozessor konfiguriert ist zum

   - Erhalten von mehreren fetalen Vektorkardiogrammen, die unterschiedlichen Herzschlägen desselben Fetus entsprechen, aus den mehreren fetalen ECG-Signalen,
   - Mitteln der mehreren fetalen VCGs, um ein gemitteltes fetales VCG zu erhalten, wobei das gemittelte fetale VCG als die Eingabe an den Maschinenlernklassifizierer bereitgestellt wird.

5. Vorrichtung zum Verarbeiten eines fECGs nach Anspruch 4, wobei der Prozessor konfiguriert ist zum

   - Bestimmen einer fetalen Bewegung aus mindestens den mehreren ECG-Signalen während eines Messzeitraums, in dem die mehreren ECG-Signale empfangen werden;
   - Rotieren der mehreren fetalen VCGs in Bezug zueinander, um die fetale Bewegung vor der Mittelung zu kompensieren.

6. Vorrichtung zum Verarbeiten eines fECGs nach einem der vorhergehenden Ansprüche, wobei der Prozessor konfiguriert ist zum

   - Detektieren fetaler QRS-Komplexe in den fetalen ECG-Signalen,
   - Verwerfen der empfangenen mehreren ECG-Signale, falls die Anzahl der detektierten fetalen QRS-Komplexe unter einem Schwellenwert liegt.

7. Vorrichtung zum Verarbeiten eines fECGs nach einem der vorhergehenden Ansprüche, wobei der Prozessor konfiguriert ist zum

   - Translatieren des fetalen VCGs, um den Mittelwert zu standardisieren, und/oder Skalieren des fetalen VCGs, um die Standardabweichung zu standardisieren, bevor das fetale VCG als Eingabe bereitgestellt wird.

8. Vorrichtung zum Verarbeiten eines fECGs nach einem der vorhergehenden Ansprüche, wobei der Prozessor konfiguriert ist zum

   - Bereitstellen mehrerer fetaler Vektorkardiogramme, die unterschiedlichen Herzschlägen desselben Fetus entsprechen, als Eingabe an den Maschinenlernklassifizierer, um mehrere Klassifizierungen zu erhalten,
   - Kommunizieren einer Klassifizierung an einen Bediener der Vorrichtung, falls mindestens eine Schwellenanzahl der mehreren Klassifizierungen diese Klassifizierung angibt.

9. Vorrichtung zum Verarbeiten eines fECGs nach einem der vorhergehenden Ansprüche, wobei der Maschinenlernklassifizierer konfiguriert ist zum Empfangen von mehreren fetalen Vektorkardiogrammen, die unterschiedlichen Herzschlägen desselben Fetus entsprechen, als Teil derselben Eingabe, wobei der Trainingssatz fetale Vektorkardiogramme umfasst, die mit fetaler Arrhythmie diagnostizierten Feten entsprechen.

10. Vorrichtung zum Verarbeiten eines fECGs nach einem der vorhergehenden Ansprüche, wobei der Prozessor konfiguriert ist zum

   - Empfangen von fetaler Orientierung, und Berechnen der Orientierung der elektrischen Herzachse in Bezug auf die fetale Orientierung, und wobei der Prozessor konfiguriert ist zum
   - Bereitstellen der Orientierung der elektrischen Herzachse an den Maschinenlernklassifizierer, oder
   - Normalisieren der Orientierung, indem das fetale VCG rotiert wird, um auf fetale Orientierung zu korrigieren.

11. Vorrichtung zum Verarbeiten eines fECGs nach einem der vorhergehenden Ansprüche, wobei das Verfahren zum Trainieren des Maschinenlernklassifizierers Erweitern des Trainingssatzes durch Hinzufügen von Rauschen zu den Trainingsdaten umfasst.

12. Vorrichtung zum Verarbeiten eines fECGs nach einem der vorhergehenden Ansprüche, wobei der Prozessor konfiguriert ist zum Berechnen einer Konfidenzschätzung für die Klassifizierung.

13. Vorrichtung zum Verarbeiten eines fECGs nach einem der vorhergehenden Ansprüche, wobei der Prozessor konfiguriert ist zum Bereitstellen von Ultraschalldaten als eine weitere Eingabe an den Maschinenlernklassifizierer.

14. Computerlesbares Medium (1000), umfassend transitorische oder nichttransitorische Daten (1020), die Anweisungen repräsentieren, um zu bewirken, dass ein Prozessorsystem Folgendes durchführt:
ein Verfahren zum Verarbeiten eines fetalen Elektrokardiogramms (fECG) zum Detektieren einer angeborenen Herzerkrankung (CHD), wobei das Verfahren umfasst

- Empfangen mehrerer ECG-Signale von mehreren Elektroden, wobei die Elektroden zum Anordnen am Bauch einer schwangeren Frau konfiguriert sind,
- Speichern trainierter Parameter für einen Maschinenlernklassifizierer zum Klassifizieren eines fetalen Vektorkardiogramms als eine CHD aufweisend oder nicht, wobei die trainierten Parameter aus einem Verfahren zum Trainieren eines Maschinenlernklassifizierers zum Klassifizieren eines fetalen Vektorkardiogramms als eine CHD aufweisend oder nicht erhalten wurden, wobei das Verfahren Trainieren des Maschinenlernklassifizierers an einem Trainingssatz von fetalen Vektorkardiogrammen umfasst, wobei der Trainingssatz fetale Vektorkardiogramme, die mit CHD diagnostizierten Feten entsprechen, und fetale Vektorkardiogramme umfasst, die nicht mit einer Herzerkrankung diagnostizierten Feten entsprechen,
- Erhalten mehrerer fetaler ECG-Signale aus den mehreren ECG-Signalen, und Berechnen mindestens eines fetalen Vektorkardiogramms (VCG) aus den mehreren fetalen ECG-Signalen,
- Bereitstellen des fetalen Vektorkardiogramms als eine Eingabe an den mit den trainierten Parametern konfigurierten Maschinenlernklassifizierer, und Erhalten einer Klassifizierung von dem Maschinenlernklassifizierer, wobei Berechnen des fetalen VCGs mit einer höheren Abtastrate durchgeführt wird, wobei das fetale Vektorkardiogramm auf eine niedrigere Abtastrate unterabgetastet wird, bevor es dem Maschinenlernklassifizierer als eine Eingabe bereitgestellt wird,
- Kommunizieren der Klassifizierung an einen Bediener der Vorrichtung.

## Revendications

1. Dispositif (200) destiné à traiter un électrocardiogramme fœtal (fECG) pour détecter une cardiopathie congénitale (CHD), le dispositif comprenant

- une entrée de signal (210) destinée à recevoir de multiples signaux d'ECG provenant de multiples électrodes (211), lesdites électrodes étant conçues pour être disposées sur l'abdomen d'une femme enceinte,
- un dispositif de stockage (220) destiné à stocker des paramètres entraînés pour un classificateur d'apprentissage automatique destiné à classer un cardiogramme vectoriel fœtal comme ayant ou non une CHD, les paramètres entraînés ayant été obtenus par un procédé d'entraînement d'un classificateur d'apprentissage automatique destiné à classer un cardiogramme vectoriel fœtal comme ayant ou non une CHD, le procédé comprenant l'entraînement du classificateur d'apprentissage automatique sur un ensemble d'entraînement de cardiogrammes vectoriels fœtaux, l'ensemble d'entraînement comprenant des cardiogrammes vectoriels fœtaux correspondant à des fœtus diagnostiqués avec une CHD et des cardiogrammes vectoriels fœtaux correspondant à des fœtus non diagnostiqués avec une cardiopathie,
- un processeur conçu pour
- obtenir de multiples signaux d'ECG fœtal à partir des multiples signaux d'ECG, et calculer au moins un cardiogramme vectoriel fœtal (VCG) à partir desdits multiples signaux d'ECG fœtal,
- fournir le cardiogramme vectoriel fœtal comme entrée au classificateur d'apprentissage automatique configuré avec les paramètres entraînés, et obtenir du classificateur d'apprentissage automatique une classification, le calcul du VCG fœtal étant effectué sur un taux d'échantillonnage supérieur, le cardiogramme vectoriel fœtal étant sous-échantillonné à un taux d'échantillonnage inférieur avant d'être fourni comme entrée au classificateur d'apprentissage automatique,
- communiquer la classification à un opérateur du dispositif.

2. Dispositif destiné à traiter un fECG selon la revendication 1, le procédé d'entraînement du classificateur d'apprentissage automatique comprenant l'entraînement du classificateur d'apprentissage automatique sur un ensemble d'entraînement de cardiogrammes vectoriels fœtaux, l'ensemble d'entraînement étant augmenté par rotation aléatoire de cardiogrammes vectoriels fœtaux avant l'entraînement pour empêcher le classificateur d'apprentissage automatique d'associer la classification à une orientation fœtale.

3. Dispositif destiné à traiter un fECG selon l'une quelconque des revendications précédentes, le classificateur d'apprentissage automatique étant un réseau neuronal profond.

4. Dispositif destiné à traiter un fECG selon l'une quelconque des revendications précédentes, le processeur étant conçu pour

   - obtenir de multiples cardiogrammes vectoriels fœtaux correspondant à des battements cardiaques différents du même fœtus à partir desdits multiples signaux d'ECG fœtal,
   - établir la moyenne des multiples VCG fœtaux pour obtenir un VCG fœtal moyen, le VCG fœtal moyen étant fourni comme entrée au classificateur d'apprentissage automatique.

5. Dispositif destiné à traiter un fECG selon la revendication 4, le processeur étant conçu pour

   - déterminer un mouvement fœtal au moins à partir des multiples signaux d'ECG sur une période de mesure au cours de laquelle les multiples signaux d'ECG sont reçus,
   - faire tourner les multiples VCG fœtaux les uns par rapport aux autres pour compenser le mouvement fœtal avant l'établissement de la moyenne.

6. Dispositif destiné à traiter un fECG selon l'une quelconque des revendications précédentes, le processeur étant conçu pour

   - détecter des complexes QRS fœtaux dans les signaux d'ECG fœtal,
   - rejeter les multiples signaux d'ECG reçus si le nombre de complexes QRS fœtaux détectés est inférieur à un seuil.

7. Dispositif destiné à traiter un fECG selon l'une quelconque des revendications précédentes, le processeur étant conçu pour

   - translater le VCG fœtal pour normaliser la moyenne et/ou mettre à l'échelle le VCG fœtal pour normaliser l'écart type avant de fournir le VCG fœtal comme entrée.

8. Dispositif destiné à traiter un fECG selon l'une quelconque des revendications précédentes, le processeur étant conçu pour

   - fournir de multiples cardiogrammes vectoriels fœtaux correspondant à des battements cardiaques différents du même fœtus comme entrée au classificateur d'apprentissage automatique afin d'obtenir de multiples classifications,
   - communiquer une classification à un opérateur du dispositif si au moins un nombre seuil des multiples classifications indique ladite classification.

9. Dispositif destiné à traiter un fECG selon l'une quelconque des revendications précédentes, le classificateur d'apprentissage automatique étant conçu pour recevoir de multiples cardiogrammes vectoriels fœtaux correspondant à des battements cardiaques différents du même fœtus comme parties de la même entrée, l'ensemble d'entraînement comprenant des cardiogrammes vectoriels fœtaux correspondant à des fœtus diagnostiqués avec une arythmie fœtale.

10. Dispositif destiné à traiter un fECG selon l'une quelconque des revendications précédentes, le processeur étant conçu pour

    - recevoir une orientation fœtale et calculer l'orientation de l'axe électrique du cœur par rapport à l'orientation fœtale, et le processeur étant conçu pour
    - fournir l'orientation de l'axe électrique du cœur au classificateur d'apprentissage automatique, ou
    - normaliser l'orientation en faisant tourner le VCG fœtal afin de corriger l'orientation fœtale.

11. Dispositif destiné à traiter un fECG selon l'une quelconque des revendications précédentes, le procédé d'entraînement du classificateur d'apprentissage automatique comprenant l'augmentation de l'ensemble d'entraînement par ajout de bruit aux données d'entraînement.

12. Dispositif destiné à traiter un fECG selon l'une quelconque des revendications précédentes, le processeur étant conçu pour calculer une estimation de confiance pour la classification.

13. Dispositif pour traiter un fECG selon l'une quelconque des revendications précédentes, le processeur étant conçu pour fournir des données ultrasonores comme entrée supplémentaire au classificateur d'apprentissage automatique.

14. Support lisible par ordinateur (1000) comprenant des données transitoires ou non transitoires (1020) représentant des instructions pour amener un système de processeurs à effectuer :
un procédé destiné à traiter un électrocardiogramme fœtal (fECG) pour détecter une cardiopathie congénitale (CHD), le procédé comprenant

   - la réception de multiples signaux d'ECG provenant de multiples électrodes, lesdites électrodes étant conçues pour être disposées sur l'abdomen d'une femme enceinte,
   - le stockage de paramètres entraînés pour un classificateur d'apprentissage automatique destiné à classer un cardiogramme vectoriel fœtal comme ayant ou non une CHD, les paramètres entraînés ayant été obtenus par un procédé d'entraînement d'un classificateur d'apprentissage automatique destiné à classer un cardiogramme vectoriel fœtal comme ayant ou non une CHD, le procédé comprenant l'entraînement du classificateur d'apprentissage automatique sur un ensemble d'entraînement de cardiogrammes vectoriels fœtaux, l'ensemble d'entraînement comprenant des cardiogrammes vectoriels fœtaux correspondant à des fœtus diagnostiqués avec une CHD et des cardiogrammes vectoriels fœtaux correspondant à des fœtus non diagnostiqués avec une cardiopathie,
   - l'obtention de multiples signaux d'ECG fœtal à partir des multiples signaux d'ECG, et le calcul d'au moins un cardiogramme vectoriel fœtal (VCG) à partir desdits multiples signaux d'ECG fœtal,
   - la fourniture du cardiogramme vectoriel fœtal comme entrée au classificateur d'apprentissage automatique configuré avec les paramètres entraînés, et l'obtention du classificateur d'apprentissage automatique d'une classification, le calcul du VCG fœtal étant effectué sur un taux d'échantillonnage supérieur, le cardiogramme vectoriel fœtal étant sous-échantillonné à un taux d'échantillonnage inférieur avant d'être fourni comme entrée au classificateur d'apprentissage automatique,
   - la communication de la classification à un opérateur du dispositif.

Fig. 1a

Fig. 1b

Fig. 1c

Fig. 2a

Fig. 2b

Fig. 3

Fig. 4

*Fig. 5*

*Fig. 6*

*Fig. 7*

Fig. 8

Fig. 9a

Fig. 9b

510

500

520

530

540

550

560

570

610

600

620

630

Fig. 10

Fig. 11

*1000*

*1010*

*1020*

# Fig. 12a

*1110*

*1130*

*1120*

*1122*

*1124*

*1126*

*1140*

# Fig. 12b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **VULLINGS R et al.** *Vectorcardiographic loop alignment for fetal movement detection using the Expectation-Maximization algorithm and Support Vector Machines*
- **SUNEMARK M et al.** *Serial VCG/ECG Analysis Using Neural Networks*
- Bayesian Approach to Patient-Tailored Vectorcardiography. **VULLINGS R et al.** IEEE TRANSACTIONS ON BIOMEDICAL ENGINEERING. IEEE, March 2010, vol. 57, 586-595
- **DOLK H** ; **LOANE M** ; **GARNE E**. a European Surveillance of Congenital Anomalies (EUROCAT) Working Group. *Congenital Heart Defects in Europe: Prevalence and Perinatal Mortality, 2000 to 2005. Circulation*, 2011, vol. 123 (8), 841-9
- **VAN DER LINDE D** ; **KONINGS EEM** ; **SLAGER MA** ; **WITSENBURG M** ; **HELBING WA** ; **TAKKENBERG JJM**. Birth Prevalence of Congenital Heart Disease Worldwide. *J Am Coll Cardiol*, November 2011, vol. 58 (21), 2241-7
- **HOFFMAN JIE** ; **KAPLAN S**. The incidence of congenital heart disease. *J Am Coll Cardiol.*, 19 June 2002, vol. 39 (12), 1890-900
- **NELLE M** ; **RAIO L** ; **PAVLOVIC M** ; **CARREL T** ; **SURBEK D** ; **MEYER-WITTKOPF M**. Prenatal diagnosis and treatment planning of congenital heart defects-possibilities and limits. *World J Pediatr*, February 2009, vol. 5 (1), 18-22
- Lethal malformations and perinatal mortality: a 10 year review with comparison of ethnic differences. **YOUNG ID** ; **CLARKE M**. Br Med J Clin Res. 11 July 1987, vol. 295, 89-91
- **FESSLOVA' V** ; **BRANKOVIC J** ; **BOSCHETTO C** ; **MASINI A** ; **PRANDSTRALLER D** ; **PEROLO A**. Changed outcomes of fetuses with congenital heart disease: new Italian Multicentre study. *J Cardiovasc Med*, August 2015, vol. 16 (8), 568-75
- **RAZZAGHI H** ; **OSTER M** ; **REEFHUIS J**. Long-Term Outcomes in Children with Congenital Heart Disease: National Health Interview Survey. *J Pediatr*, January 2015, vol. 166 (1), 119-124.e1
- **CLUR SAB** ; **VAN BRUSSEL PM** ; **MATHIJSSEN IB** ; **PAJKRT E** ; **OTTENKAMP J**. Audit of 10 years of referrals for fetal echocardiography: AUDIT OF 10 YEARS OF REFERRALS FOR FETAL ECHOCARDIOGRAPHY. *Prenat Diagn*, September 2011
- **BONNET D** ; **COLTRI A** ; **BUTERA G** ; **FERMONT L** ; **LE BIDOIS J** ; **KACHANER J**. Detection of transposition of the great arteries in fetuses reduces neonatal morbidity and mortality. *Circulation*, 23 February 1999, vol. 99 (7), 916-8
- **MAHLE WT** ; **CLANCY RR** ; **MCGAURN SP** ; **GOIN JE** ; **CLARK BJ**. Impact of prenatal diagnosis on survival and early neurologic morbidity in neonates with the hypoplastic left heart syndrome. *Pediatrics*, June 2001, vol. 107 (6), 1277-82
- **TWORETZKY W** ; **MCELHINNEY DB** ; **REDDY VM** ; **BROOK MM** ; **HANLEY FL** ; **SILVERMAN NH**. Improved surgical outcome after fetal diagnosis of hypoplastic left heart syndrome. *Circulation*, 06 March 2001, vol. 103 (9), 1269-73
- **FRANKLIN O** ; **BURCH M** ; **MANNING N** ; **SLEEMAN K** ; **GOULD S** ; **ARCHER N**. Prenatal diagnosis of coarctation of the aorta improves survival and reduces morbidity. *Heart Br Card Soc*, January 2002, vol. 87 (1), 67-9
- **MÄKIKALLIO K** ; **MCELHINNEY DB** ; **LEVINE JC** ; **MARX GR** ; **COLAN SD** ; **MARSHALL AC**. Fetal aortic valve stenosis and the evolution of hypoplastic left heart syndrome: patient selection for fetal intervention. *Circulation*, 21 March 2006, vol. 113 (11), 1401-5
- **KAGUELIDOU F** ; **FERMONT L** ; **BOUDJEMLINE Y** ; **LE BIDOIS J** ; **BATISSE A** ; **BONNET D**. Foetal echocardiographic assessment of tetralogy of Fallot and post-natal outcome. *Eur Heart J.*, 18 January 2008, vol. 29 (11), 1432-8
- **TRINES J** ; **FRUITMAN D** ; **ZUO KJ** ; **SMALLHORN JF** ; **HORNBERGER LK** ; **MACKIE AS**. Effectiveness of Prenatal Screening for Congenital Heart Disease: Assessment in a Jurisdiction With Universal Access to Health Care. *Can J Cardiol.*, July 2013, vol. 29 (7), 879-85
- **HUNTER LE** ; **SIMPSON JM**. Prenatal screening for structural congenital heart disease. *Nat Rev Cardiol.*, June 2014, vol. 11 (6), 323-34
- **VAN VELZEN CL** ; **HAAK MC** ; **REIJNDERS G** ; **RIJLAARSDAM MEB** ; **BAX CJ** ; **PAJKRT E**. Prenatal detection of transposition of the great arteries reduces mortality and morbidity. *Ultrasound Obstet Gynecol*, March 2015, vol. 45 (3), 320-5

- **BROWN KL** ; **RIDOUT DA** ; **HOSKOTE A** ; **VERHULST L** ; **RICCI M**. Delayed diagnosis of congenital heart disease worsens preoperative condition and outcome of surgery in neonates. *Heart Br Card Soc.*, September 2006, vol. 92 (9), 1298-302
- **VAN VELZEN C** ; **CLUR S** ; **RIJLAARSDAM M** ; **BAX C** ; **PAJKRT E** ; **HEYMANS M**. Prenatal detection of congenital heart disease-results of a national screening programme. *BJOG Int J Obstet Gynaecol*, February 2016, vol. 123 (3), 400-7
- **LEVY DJ** ; **PRETORIUS DH** ; **ROTHMAN A** ; **GONZALES M** ; **RAO C** ; **NUNES ME**. Improved Prenatal Detection of Congenital Heart Disease in an Integrated Health Care System. *Pediatr Cardiol*, March 2013, vol. 34 (3), 670-9
- **MAREK J** ; **TOMEK V** ; **SKOVRANEK J** ; **POVYSILOVA V** ; **SAMANEK M**. Prenatal ultrasound screening of congenital heart disease in an unselected national population: a 21-year experience. *Heart*, 15 January 2011, vol. 97 (2), 124-30
- **BUSKENS E** ; **GROBBEE DE** ; **FROHN-MULDER IM** ; **STEWART PA** ; **JUTTMANN RE** ; **WLADIMIROFF JW**. Efficacy of routine fetal ultrasound screening for congenital heart disease in normal pregnancy. *Circulation*, 01 July 1996, vol. 94 (1), 67-72
- **SHARLAND GK** ; **ALLAN LD**. Screening for congenital heart disease prenatally. Results of a 2 1/2-year study in the South East Thames Region. *Br J Obstet Gynaecol*, March 1992, vol. 99 (3), 220-5
- **STÜMPFLEN I** ; **STÜMPFLEN A** ; **WIMMER M** ; **BERNASCHEK G**. Effect of detailed fetal echocardiography as part of routine prenatal ultrasonographic screening on detection of congenital heart disease. *Lancet Lond Engl.*, 28 September 1996, vol. 348 (9031), 854-7
- **GALINDO A** ; **HERRAIZ I** ; **ESCRIBANO D** ; **LORA D** ; **MELCHOR JC** ; **DE LA CRUZ J**. Prenatal Detection of Congenital Heart Defects: A Survey on Clinical Practice in Spain. *Fetal Diagn Ther*, 2011, vol. 29 (4), 287-95
- **VAN LAAR J** ; **WARMERDAM G** ; **VERDURMEN K** ; **VULLINGS R** ; **PETERS C** ; **HOUTERMAN S** ; **WIJN P** ; **ANDRIESSEN P** ; **VAN PUL C** ; **OEI G.** Fetal heart rate variability during pregnancy, obtained from non-invasive electrocardiogram recordings. *Acta Obstet Gynecol Scand.*, 2014, vol. 93, 93-101
- **VERDURMEN K** ; **LEMPERSZ C** ; **VULLINGS R** ; **SCHROER C** ; **DELHAAS T** ; **VAN LAAR J** ; **OEI S**. Normal ranges for fetal electrocardiogram values for the healthy fetus of 18-24 weeks of gestation: a prospective cohort study. *BMC Pregnancy Childbirth.*, 2016, vol. 16, 227
- **VULLINGS R** ; **PETERS CHL** ; **SLUIJTER RJ** ; **MISCHI M** ; **OEI SG** ; **BERGMANS JWM**. Dynamic segmentation and linear prediction for maternal ECG removal in antenatal abdominal recordings. *Physiol Meas*, 01 March 2009, vol. 30 (3), 291-307
- **VULLINGS R** ; **PETERS CHL** ; **MOSSAVAT I** ; **OEI SG** ; **BERGMANS JWM**. Bayesian Approach to Patient-Tailored Vectorcardiography. *IEEE Trans Biomed Eng.*, March 2010, vol. 57 (3), 586-95
- **WARMERDAM GJJ** ; **VULLINGS R** ; **SCHMITT L** ; **VAN LAAR JOEH** ; **BERGMANS JWM**. A Fixed-Lag Kalman Smoother to Filter Power Line Interference in Electrocardiogram Recordings. *IEEE Trans Biomed Eng.*, August 2017, vol. 64 (8), 1852-61
- **WARMERDAM GJJ** ; **VULLINGS R** ; **SCHMITT L** ; **VAN LAAR JOEH** ; **BERGMANS JWM**. Hierarchical Probabilistic Framework for Fetal R-Peak Detection, Using ECG Waveform and Heart Rate Information. *IEEE Trans Signal Process.*, 15 August 2018, vol. 66 (16), 4388-97
- **FOTIADOU E** ; **VAN LAAR JOEH** ; **OEI SG** ; **VULLINGS R**. Enhancement of low-quality fetal electrocardiogram based on time-sequenced adaptive filtering. *Med Biol Eng Comput [Internet].*, 25 June 2018, http://link.springer.com/10.1007/s11517-018-1862-8
- **VULLINGS R** ; **MISCHI M** ; **OEI SG** ; **BERGMANS JWM**. Novel Bayesian Vectorcardiographic Loop Alignment for Improved Monitoring of ECG and Fetal Movement. *IEEE Trans Biomed Eng.*, June 2013, vol. 60 (6), 1580-8
- **VERDURMEN KMJ** ; **HULSENBOOM ADJ** ; **VAN LAAR JOEH** ; **WIJN PFF** ; **VULLINGS R** ; **OEI SG**. Orientation of the electrical heart axis in mid-term pregnancy. *Eur J Obstet Gynecol Reprod Biol.*, December 2016, vol. 207, 243-6
- **FOTIADOU**. *Enhancement of low-quality fetal electrocardiogram based on time-sequenced adaptive filtering*